(19) Europäisches Patentamt
European Patent Office
Office européen des brevets

(11) EP 1 588 692 B1

(12) **EUROPÄISCHE PATENTSCHRIFT**

(45) Veröffentlichungstag und Bekanntmachung des Hinweises auf die Patenterteilung:
**24.09.2008 Patentblatt 2008/39**

(51) Int Cl.:
*A61Q 5/06* (2006.01)          *A61Q 5/00* (2006.01)
*A61K 8/894* (2006.01)

(21) Anmeldenummer: **05003965.0**

(22) Anmeldetag: **24.02.2005**

(54) **Dauerhaft geschäumte Zusammensetzung**

Durable foamed composition

Composition sous forme de mousse durable

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **05.04.2004 DE 102004016683**

(43) Veröffentlichungstag der Anmeldung:
**26.10.2005 Patentblatt 2005/43**

(73) Patentinhaber: **Wella Aktiengesellschaft
64274 Darmstadt (DE)**

(72) Erfinder:
• **Baumeister, Jan, Dr.
1726 Farvagny-le-Grand (CH)**
• **Maillefer, Sarah, Dr.
1749 Middes (CH)**
• **Rehmann, Andre
3185 Schmitten (CH)**

(56) Entgegenhaltungen:
**EP-A- 0 838 210          EP-A- 1 078 626
EP-A- 1 149 872          EP-A- 1 369 104
WO-A-02/41847          WO-A-03/028676
WO-A-20/04039338**

**Beschreibung**

Gegenstand der Erfindung sind dauerhaft mit einem Gas gemäß Anspruch 1 aufgeschäumte Zusammensetzungen einer Dichte von kleiner oder gleich 0,8 g/cm$^3$ auf wässriger Basis unter Verwendung von bestimmten, ausgewählten alkoxylierten Silikonverbindungen und vorzugsweise zusätzlichen Konsistenzgebern.

Kosmetische Produkte unterliegen besonderen Anforderungen hinsichtlich der dem Anwender übermittelten sensorischen Eigenschaften. Haut- und Haarpflegeprodukte wie Hautcremes oder Haarkurmittel liegen häufig in Form von cremigen Emulsionen oder Dispersionen vor und bestehen aus einer wässrigen, hydrophilen Phase und aus einer Öl- oder Wachsstoffe enthaltenden hydrophoben Phase. Nachteilig an diesen herkömmlichen emulsions- bzw. dispersionsförmigen kosmetischen Pflegeprodukten ist deren Sensorik, insbesondere deren Haptik. Die Produkte fühlen sich häufig zu schwer, fettig und schmierig an. Viele Cremes haben aufgrund der für ihre Wirksamkeit erforderlichen Bestandteile ein unvorteilhaftes, beigefarbenes oder gelbstichiges Aussehen. Bei Haarkurmitteln und bei Shampoos ist zusätzlich eine verbesserte Verteilbarkeit auf dem Haar und ein verbessertes Aufemulgieren bei einer Einarbeitung in feuchtes Haar wünschenswert. Haarstylingmittel liegen häufig in Form von Verdickern enthaltenden gelförmigen Produken mit unvorteilhaftem, klebrigen Anfühlen vor. Im schnelllebigen Haarstylingmarkt ist der Bedarf an Produkten mit neuen Eigenschaften sehr groß. Hohes Aufmerksamkeitspotential haben Produktinnovationen, die die mehrere Sinne gleichzeitig ansprechen und positive Assoziationen wecken.

Kosmetische Haarbehandlungsprodukte in Form von unmittelbar vor der Anwendung aufgeschäumten temporären, instabilen Schäumen sind bekannt. Es handelt sich dabei entweder um Aerosol-Produkte, welche bei der Entnahme aus einem Druckbehälter mittels eines Treibgases zu einem instabilen Schaum aufgeschäumt werden oder es handelt sich um sogenannte Pumpschäume, welche bei der Entnahme aus der Verpackung mittels einer mechanischen Pumpe in Verbindung mit einem Schaumkopf einen instabilen Schaum produzieren. Derartige, temporäre, innerhalb weniger Minuten zusammenbrechende Schäume bildenden Produkte haben den Nachteil, dass der Schaum vor jeder Anwendung erst noch hergestellt werden muss, was für den Anwender umständlich ist. Ausserdem sind die benötigten Verpackungen aufwändig und fehleranfällig, da die Gefahr von Verstopfungen der Produktabgabesysteme besteht. Zudem ist die Rezeptierungsfreiheit eingeschränkt, d.h. nicht alle gewünschten Haut- und Haarpflegewirkungen sind mit temporären Schaumprodukten realisierbar.

EP 838 210 A2 offenbart die Verwendung mindestens einer wasserunlöslichen synthetischen oder natürlichen Faserart oder deren Gemische zur Herstellung eines kosmetischen Mittels zur Behandlung, Gestaltung oder Pflege der Frisur.

EP 1 078 626 A2 bezieht sich auf eine Zusammensetzung zur Reparatur splissiger Haarenden. Die Zusammensetzung enthält Guar, eine Betainbasierendes Polyurethan-Netzmittel sowie ein Silikon-Polyurethan.

In EP 1 369 104 A1 wird eine einphasige kosmetische Zubereitung zur Haarpflege offenbart, die auf Wasser, Alkohol oder Öl basiert und Alkylmethylsiloxan-dimethylsiloxan-polyalkylen-oxid-Copolymere enthält.

WO 03/028676 A2 offenbart selbstschäumende, schaumförmige, nachschäumende oder schäumbare kosmetische und dermatologische Zubereitungen, welche mindestens ein Wachs und/oder ein bei Raumtemperatur festes oder halbfestes Lipid enthalten.

EP 1 149 872 A2 bezieht sich auf Alkylmethylsiloxan-dimethylsiloxan-polyalkylen-oxid-Copolymere, die sich als Netzmittel sowohl für Öl in Wasser Emulsionen als auch für Silikon in Wasser Emulsionen eignen.

Verschiedene Ansätze zur Herstellung von dauerhaft geschäumten kosmetischen Fertigpräparaten sind bekannt, z.B. aus WO 02/41847, WO 02/67882, EP 1 046 387. EP 1 216 682, CH 674 804 oder JP 56-79613. Die Herstellung von auch bei erhöhten Temperaturen besonders lagerstabilen, geschäumten Produkten mit gleichzeitig guten Anwendungseigenschaften bei der Anwendung auf Haut oder Haaren ist aber noch nicht in allen Punkten zufriedenstellend gelöst. Es bestand daher die Aufgabe, leicht anwendbare kosmetische Haut- und Haarpflegemittel mit verbesserten sensorischen Eigenschaften (z.B. Haptik, Optik, Akustik) zur Verfügung zu stellen, ohne dabei die primären, haut- bzw. haarpflegenden Eigenschaften unakzeptabel zu beeinträchtigen, wobei die Mittel keine aufwändigen, fehleranfälligen Verpackungen erfordern sollen und bei hoher Lagerstabilität in dauerhaft geschäumter Form vorliegen.

Gelöst wird die Aufgabe durch die Verwendung von bestimmten Silikonverbindungen als Schaumstabilisatoren für Permanentschäume. Gegenstand der Erfindung ist eine aufgeschäumte gemäß Anspruch 1 Zusammensetzung enthaltend Wasser und mindestens eine alkoxylierte Silikonverbindung, welche zu einer oder mehreren der folgenden Verbindungsklassen gehört:

- bis-alkoxylierte Silikonverbindungen
- alkoxylierte Silikonwachse
- Ester aus Fettsäuren und alkoxylierten Silikonverbindungen
- wasserunlöslichen alkoxylierten Silikonverbindungen,

wobei die Zusammensetzung mit Luft oder einem inerten Gas aufgeschäumt ist und dauerhaft eine Dichte von kleiner oder gleich 0,8 g/cm$^3$ aufweist.

Die Zusammensetzungen enthalten

(A) mindestens eine der oben genannten alkoxylierten Silikonverbindungen,
(B) mindestens einen Konsistenzgeber, ausgewählt aus bei 25°C wachsartig-festen Stoffen und Verdickern und
(C) Wasser.

Mit den erfindungsgemäßen Zusammensetzungen lassen sich kosmetische Haut- oder Haarbehandlungsmittel herstellen, welche als fertig präparierte Schaumprodukte in einer geeigneten Verpackung vorliegen und dieser entnommen werden können, wobei der Aufschäumgrad nach Lagerung von mindestens einer Woche bei Raumtemperatur (20°C) noch mindestens 10% oder mehr beträgt . Die Konsistenz der Zusammensetzung kann dabei fest, halbfest oder cremig sein.

Es handelt sich um Produkte mit polysensuellen Wirkungen. Mit dem Begriff "Polysensualität" lassen sich die Eindrücke überschreiben ähnlichen den von *mousse au chocolat* vermittelten Eindrücken. Hand, Haut und Ohr empfinden die Leichtigkeit einer aufgeschäumten Masse, die erhöhte Cremigkeit bei leichterer Verreibbarkeit und das leise Knistern der stabil geschäumten Masse bei Entnahme und Anwendung. Die reduzierte Dichte erlaubt eine genauere Dosierung und eine bessere Verteilbarkeit als herkömmliche Produkte wie Wachse, Gele oder Cremes.

Neben Wasser können in einer hydrophilen Phase weitere, wasserlösliche, kosmetisch verträgliche, organische Lösungsmittel in Mengen von 1 bis 30 Gew.% oder 5 bis 20 Gew.% enthalten sein. Derartige Lösungsmittel sind z.B. niedere einwertige Alkohole wie Ethanol oder Isopropanol oder mehrwertige C2- bis C4-Alkohole wie z.B. Ethylenglykol, Diethylenglykol, Butylenglykol oder Glycerin.

Alkoxylierte Silikonverbindungen

Die alkoxylierten Silikonverbindungen sind vorzugsweise in einer Menge von 0,1 bis 30 Gew.%, oder von 0,2 bis 20 Gew.%, besondes bevorzugt von 1 bis 10 Gew.%, bezogen auf die ungeschäumte Zusammensetzung, enthalten. Alkoxylierte Silikonverbindungen sind polyalkoxylierte Silikonverbidnungen, d.h. sie weisen Polyalkylenoxidgruppen auf. Silikonwachse sind Stoffe, welche bei Raumtemperatur wachsartig fest sind, d.h. einen Schmelz- oder Tropfpunkt von größer 25°C aufweisen. Bei den Fettsäuren handelt es sich vorzugsweise um Carbonsäuren, Dicarbonsäuren oder Hydroxycarbonsäuren mit 8 bis 32 C-Atomen. Wasserunlösliche Verbindungen sind Stoffe, welche sich bei 25°C zu vorzugsweise weniger als 1 Gew.% in reinem Wasser lösen.

Geeignete bis-alkoxylierte Silikonverbindungen sind Poly-(dialkylsiloxane), welche zwei end- oder seitenständige Polyoxyalkylenketten aufweisen. Bevorzugt sind Blockcopolymere, insbesondere vom Typ ABA, mit einem Mittelblock aus Polydimethylsiloxan und Endblöcken aus Polyethylenoxid und/oder Polypropylenoxid. Die Endblöcke können endständig unsubstituiert sein, d.h. Hydroxylgruppen aufweisen oder sie können endständig substituiert sein, z.B. mit Ether-, Ester- oder Urethangruppen, insbesondere Fettsäureester. Der Alkoxylierungsgrad ist vorzugsweise von 2 bis 40, insbesondere von 10 bis 30, besonders bevorzugt von 12 bis 20.

Geeignete Silikonverbindungen sind solche der allgemeinen Formel

$$R1\text{-}(AO)_{x1}\text{-}B1\text{-}SiMe_2O\text{-}(SiMe_2O)_{x3}\text{-}SiMe_2O\text{-}B2\text{-}(AO)_{x2}\text{-}R2,$$

wobei R1 eine Hydroxygruppe, eine Alkoxygruppe mit 1 bis 22 C-Atomen oder eine Carboxyalkylgruppe mit 2 bis 22 C-Atomen ist, B1 und B2 verschieden oder vorzugsweise gleich sind und eine Einfachbindung oder eine divalente Verbindungsgruppe, insbesondere eine Alkylengruppe mit 1, 2, 3 oder 4 C-Atomen bedeutet; AO eine Oxyalkylengruppe, insbesondere Oxyethylen oder Oxypropylen bedeutet; R2 Wasserstoff oder eine mit der benachbarten Oxyalkylengruppe veretherte oder veresterte Alkylgruppe mit 1 bis 22 C-Atomen ist; x1 und x2 Zahlen größer gleich 1 sind und deren Summe den Alkoxylierungsgrad angibt und x3 eine Zahl größer gleich 1 ist und den Polymerisationsgrad des Dimethylpolysiloxans angibt.

Bevorzugte Silikonverbindungen sind diejenigen mit den INCI-Bezeichnungen Bis-PEG-4 Dimethicone, Bis-PEG-12 Dimethicone, Bis-PEG-20 Dimethicone, Bis-PEG-12 Dimethicone Beeswax, Bis-PEG-12 Dimethicone Candellilate, Bis-PEG-15 Dimethicone/IPDI Copolymer, Bis-PEG-15 Methyl Ether Dimethicone, Bis-PEG-18 Methyl Ether Dimethylsilane, Bis-PEG/PPG-14/14 Dimethicone, Bis-PEG/PPG-20/20 Dimethicone, Bis-PEG/PPG-16/16 PEG/PPG-16/16 Dimethicone, Bis-PPG-15 Dimethicone/IPDI Copolymer, Bis-PPG-7 Undeceneth-21 Dimethicone. Bevorzugt sind insbesondere Bis-(polyethylenoxid)-polydimethylsiloxane, Fettsäureester von Bis-(polyethylenoxid)polydimethylsiloxan und eine Mischung aus mindestens einem Bis-(polyethylenoxid)-polydimethylsiloxan und mindestens einem Fettsäureester von Bis-(polyethylenoxid)-polydimethylsiloxan. Besonders bevorzugt sind mit Fettsäuren veresterte, bis-ethoxylierte Silikonwachse.

Weitere geeignete Silikonverbindungen sind ethoxylierte Dimethylsilanmethylether, z.B. wasserdispergierbare SilikonWachse der allgemeinen Formel

CH3-(OCH2CH2)n-O-Si(CH3)2-O-(CH2CH2O)m-CH3

worin n und m gleich oder verschieden sein können, den Ethoxylierungsgrad angeben und vorzugsweise Zahlen zwischen 5 und 40, insbesondere von 10 bis 20 bedeuten. Ein Beispiel ist die Verbindung mit der INCI-Bezeichnung Bis-PEG-18 Methyl Ether Dimethyl Silane, welches als Dow Corning® 2501 Cosmetic Wax erhältlich ist.

Wachsstoff

Der wachsartige Feststoff der Komponente (B) ist, wenn er in der Zusammensetzung enthalten ist, vorzugsweise in einer Menge von 0,05 bis 50 Gew.%, oder von 0,2 bis 30 Gew.%, besonders bevorzugt von 0,5 bis 10 Gew.%, bezogen auf die ungeschäumte Zusammensetzung, enthalten. Geeignete wachsartige Stoffe weisen einen Erstarrungspunkt auf, welcher bei größer oder gleich 25°C, vorzugsweise im Bereich von 30 bis 100°C, insbesondere im Bereich von 40 bis 90°C liegt.

Wachsartige Stoffe sind z.B. tierische Wachse, pflanzliche Wachse, mineralische Wachse, synthetische Wachse, mikrokristalline Wachse, makrokristalline Wachse, Paraffinwachse, Ozokerit, Montanwachse, Fischer-Tropsch-Wachse, Polyolefinwachse (z.B. Polyethylen, Polybuten und ähnliche), Amidwachse, Silikonwachse, Bienenwachs, Wollwachs (Lanolin) und dessen Derivate wie z.B. Wollwachsalkohole (Lanolinalkohole), Candelillawachs, Carnaubawachs, Japanwachs, Fette, Fettsäureester, Fettsäureglyceride, langkettige Carbonsäuren oder langkettige (C10- bis C22-) Alkohole, jeweils mit Schmelz- bzw. Erstarrungspunkten oberhalb 25°C. Geeignet ist z.B. Rizinuswachs, wobei es sich um gehärtetes, d.h. hydriertes Rizinusöl handelt (INCI: Hydrogenated Castor Oil) mit Schmelzpunkt bis zu etwa 90°C.

Die Zusammensetzung kann zusätzlich in einer hydrophoben Phase bei Raumtemperatur flüssige hydrophobe Stoffe enthalten. Hierbei kann es sich um Öle oder ölartige Stoffe handeln, z.B. um natürlich vorkommende, nachwachsende Öle (pflanzliche und tierische fette Öle), synthetische Öle, Silikonöle, Mineralöle, etherische Öle, wasserunlösliche, verzweigte oder lineare aliphatische Kohlenwasserstoffe, lineare oder verzweigte Alkohole, insbesondere flüssige Fettalkohole sowie langkettige Ether oder Ester, wobei die genannten Substanzen vorzugsweise mindestens 8 C-Atome aufweisen. Geeignete Kohlenwasserstoffe sind z.B. flüssige Paraffine, Squalan oder Squalene. Weiterhin geeignet sind Ester von drei- und mehrwertigen Alkoholen, insbesondere pflanzlichen Triglyceriden wie z.B. Olivenöl, Mandelöl, Erdnußöl, Sonnenblumenöl sowie synthetische Triglyceride wie z.B. C8-C10-Trifettsäureglycerinester oder auch Jojobaöl.

Weiterhin als hydrophobe Substanz geeignet sind Mono- oder Diester der Formeln $R^1$-COOR$^2$, $R^1$-COO-$R^3$-OOCR$^1$ und $R^2$OOC-$R^3$-COOR$^2$, wobei $R^1$ für eine C8- bis C22-Alkylgruppe, $R^2$ für eine C3- bis C22-Alkylgruppe und $R^3$ für eine C2- bis C16-Alkylengruppe steht. Geeignet sind auch natürlich vorkommende Monoester- bzw. Wachsestergemische, wie sie z.B. in Jojobaöl oder Spermöl vorliegen und verzweigte primäre Alkohole, wie sie unter der Bezeichnung Guerbetalkohole bekannt sind.

Als hydrophobe Substanzen sind ausserdem Stoffe geeignet, die üblicherweise als Trübungsmittel in kosmetischen Mitteln eingesetzt werden, insbesondere solche der Formel $R^1$-COO-(CHR$^4$CHR$^5$O)$_n$-COR$^6$, wobei $R^1$ für eine C8- bis C22-Alkylgruppe, $R^4$ und $R^5$ für Wasserstoff oder Methyl und $R^6$ für Wasserstoff oder für $R^1$ steht und n eine Zahl zwischen 1 und 12, vorzugsweise 1, 2, 3 oder 4 bedeutet. Bevorzugt sind Glykoldifettsäureester und Polyethylenglykoldifettsäureester, welche bei Raumtemperatur in fester Form vorliegen.

Verdicker

Verdicker der Komponente (B) sind, wenn sie in der Zusammensetzung enthalten sind, vorzugsweise in einer Menge von 0,05 bis 30 Gew.%, von 0,2 bis 20 Gew.% oder besonders bevorzugt von 0,5 bis 10 Gew.%, bezogen auf die ungeschäumte Zusammensetzung, enthalten. Geeignete Verdicker sind insbesondere solche, welche der Zusammensetzung eine Fließgrenze verleihen. Geeignete Verdicker sind

- synthetische Polymere wie z.B. Polyvinylpyrrolidon oder vernetzte Polyacrylate (Carbomere, Carbopole),
- Polymere auf natürlicher Basis, insbesondere Polysaccharide und deren Derivate, z.B. Sclerotium Gum, Stärke, Gelatine, Cellulose und deren Derivate wie Carboxymethylcellulose, Hydroxypropylcellulose, Methylcellulose, Hydroxypropylmethylcellulose oder Hydroxyethylcellulose, mikrokristalline Cellulose, sowie Extrakte aus Algen wie Agar-agar, Carrageenane oder Alginate, sowie Carouba Gum, Guar Gum und dessen Derivate wie z.B. alkylierte oder hydroxyalkyliertes Guar, Karaya Gum, Xanthan Gum, Gum arabicum, Pektine
- Anorganische Verdicker wie Hectorite, Bentonite, Aluminium- und Magnesiumsilikate

oder ein Gemisch der genannten Substanzen.

Bevorzugte Verdicker sind vernetzte Polyacrylsäuren bzw. deren Salze, Polysaccharide, Polysaccharidderivate, Agar-agar. Besonders bevorzugt ist ein Gehalt von 0,5 bis 10 Gew.% Carrageenan, insbesondere kappa-Carrageenan und/oder iota-Carrageenan.

Haarfestigende und haarpflegende Polymere

In einer Ausführungsform enthält die erfindungsgemäße Zusammensetzung mindestens ein haarfestigendes und/oder mindestens ein haarpflegendes Polymer. Die haarfestigenden oder haarpflegenden Polymere sind vorzugsweise in einer Menge von 0,01 bis 20 Gew.% oder von 0,05 bis 10 Gew.%, besonders bevorzugt von 0,1 bis 5 Gew.% enthalten. Es kann sich um anionische Polymere, d.h. um Polymere mit anionischen oder anionisierbaren Gruppen, um kationische Polymere, d.h. um Polymere mit kationischen oder kationisierbaren Gruppen, um zwitterionische Polymere, d.h. um Polymere mit kationischen und anionischen Gruppen, um amphotere Polymere, d.h. um Polymere mit sauren und basischen Gruppen oder um nichtionische Polymere handeln. Unter anionisierbaren Gruppen werden Säuregruppen wie z.B. Carbonsäure-, Sulfonsäure- oder Phosphorsäuregruppen verstanden, welche mittels üblicher Basen wie z.B. organischer Amine oder Alkali- oder Erdalkalihydroxide deprotoniert werden können.

Anionische Polymere können teilweise oder vollständig mit einem basischen Neutralisationsmittel neutralisiert sein. Bevorzugt ist ein Neutralisationsgrad von 50 bis 100 %, besonders bevorzugt von 70-100%. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH, KOH u.a..

Das anionische Polymer kann ein Homo- oder Copolymer mit Säuregruppen enthaltenden Monomereinheiten auf natürlicher oder synthetischer Basis sein, welches gegebenenfalls mit Comonomeren, die keine Säuregruppen enthalten, copolymerisiert ist. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Maleinsäuremonoester, insbesondere die Mono-C1-C7-alkylester der Maleinsäure sowie Aldehydocarbonsäuren oder Ketocarbonsäuren. Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete Polymere mit Säuregruppen sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind:

Terpolymere aus Acrylsäure, Alkylacrylat und N-Alkylacrylamid (INCI-Bezeichnung: Acrylates/Acrylamide Copolymer), insbesondere Terpolymere aus Acrylsäure, Ethylacrylat und N-tert-Butylacrylamid; vernetzte oder unvernetzte Vinylacetat/Crotonsäure Copolymere (INCI-Bezeichnung: VA/Crotonates Copolymer); Copolymere aus ein oder mehreren C1-C5-Alkylacrylaten, insbesondere C2-C4-Alkylacrylaten und mindestens einem Monomer ausgewählt aus Acrylsäure oder Methacrylsäure (INCI-Bezeichnung: Acrylates Copolymer), z.B. Terpolymere aus tert.-Butylacrylat, Ethylacrylat und Methacrylsäure; Natriumpolystyrolsulfonat; Vinylacetat/Crotonsäure/ Vinylalkanoat Copolymere, z.B. Copolymere aus Vinylacetat, Crotonsäure und Vinylpropionat; Copolymere aus Vinylacetat, Crotonsäure und Vinylneodecanoat (INCI-Bezeichnungen: VA/Crotonates/Vinyl Propionate Copolymer, VA/Crotonates/Vinyl Neodecanoate Copolymer); Aminomethylpropanol-Acrylat Copolymere; Copolymere aus Vinylpyrrolidon und mindestens einem weiteren Monomer ausgewählt aus Acrylsäure und Methacrylsäure sowie ggf. Acrylsäureestern und Methacrylsäureestern; Copolymere aus Methylvinylether und Maleinsäuremonoalkylestern (INCI-Bezeichnungen: Ethylester of PVM/MA Copolymer, Butylester of PVM/MA Copolymer); Aminomethylpropanolsalze von Copolymeren aus Allylmethacrylat und mindestens einem weiteren Monomer ausgewählt aus Acrylsäure, und Methacrylsäure sowie ggf. Acrylsäureestern und Methacrylsäureestern; vernetzte Copolymere aus Ethylacrylat und Methacrylsäure; Copolymere aus Vinylacetat, Mono-n-butylmaleat und Isobornylacrylat; Copolymere aus zwei oder mehr Monomeren ausgewählt aus Acrylsäure und Methacrylsäure sowie ggf. Acrylsäureestern und Methacrylsäureestern; Copolymere aus Octylacrylamid und mindestens einem Monomeren ausgewählt aus Acrylsäure und Methacrylsäure soweit ggf. Acrylsäureestern und Methacrylsäureestern; Polyester aus Diglycol, Cyclohexandimethanol, Isophtalsäure und Sulfoisophtalsäure, wobei die Alkylgruppen der vorstehend genannten Polymere in der Regel vorzugsweise 1, 2, 3 oder 4 C-Atome aufweisen.

Bevorzugte zwitterionische oder amphotere Polymere sind: Copolymere gebildet aus Alkylacrylamid, Alkylaminoalkylmethacrylat und zwei oder mehr Monomeren aus Acrylsäure und Methacrylsäure sowie ggf. deren Estern, insbesondere Copolymere aus Octylacrylamid, Acrylsäure, Butylaminoethylmethacrylat, Methylmethacrylat und Hydroxypropylmethacrylat (INCI-Bezeichnung: Octylacrylamide/Acrylates/Butylaminoethyl Methacrylate Copolymer); Copolymere, welche gebildet sind aus mindestens einer ersten Monomerart, welche quaternäre Amingruppen aufweist und mindestens einer zweiten Monomerart, welche Säuregruppen aufweist; Copolymere aus Fettalkoholacrylaten, Alkylaminoxidmethacrylat und mindestens einem Monomeren ausgewählt aus Acrylsäure und Methacrylsäure sowie ggf. Acrylsäureestern und Methacrylsäureestern, insbesondere Copolymere aus Laurylacrylat, Stearylacrylat, Ethylaminoxidmethacrylat und mindestens einem Monomeren ausgewählt aus Acrylsäure und Methacrylsäure sowie ggf. deren Estern; Copolymere aus Methacryloylethylbetain und mindestens einem Monomeren ausgewählt aus Methacrylsäure und Methacrylsäureestern; Copolymere aus Acrylsäure, Methylacrylat und Methacrylamidopropyltrimethylammoniumchlorid (INCI-Bezeichnung: Polyquaternium-47); Copolymere aus Acrylamidopropyltrimethylammoniumchlorid und Acrylaten oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43); Oligomere oder Polymere, herstellbar aus quaternären Crotonbetainen oder quaternären Crotonbetainestern.

Kationische Polymere sind insbesondere solche mit primären, sekundären, tertiären oder quaternären Amingruppen. Die kationische Ladungsdichte beträgt vorzugsweise 1 bis 7 meq/g. Geeignete kationische Polymere enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie beispielsweise Silikonpolymere mit quaternären Endgruppen (Quaternium-80).

Bevorzugte kationische Polymere auf synthetischer Basis:
Poly(dimethyldiallylammoniumchlorid); Copolymere aus Acrylamid und Dimethyldiallylammoniumchlorid; quaternäre Ammoniumpolymere, gebildet durch die Reaktion von Diethylsulfat und einem Copolymer aus Vinylpyrrolidon und Dimethylaminoethylmethacrylat, insbesondere Vinylpyrrolidon/Dimethylaminoethylmethacrylatmethosulfat Copolymer (z.B. Gafquat® 755 N, Gafquat® 734); quaternäre Ammoniumpolymere aus Methylvinylimidazoliumchlorid und Vinylpyrrolidon (z.B. LUVIQUAT® HM 550); Polyquaternium-35; Polyquaternium-57; Polymer aus Trimethylammonium-ethylmethacrylatchlorid; Terpolymere aus Dimethyldiallylammoniumchlorid, Natriumacrylat und Acrylamid (z.B. Merquat® Plus 3300); Copolymere aus Vinylpyrrolidon, Dimethylaminopropylmethacrylamid und Methacryloylaminopropyllauryldimethylammoniumchlorid; Terpolymere aus Vinylpyrrolidon, Dimethylaminoethylmethacrylat und Vinylcaprolactam (z.B. Gaffix® VC 713); Vinylpyrrolidon/Methacrylamidopropyltrimethylammoniumchlorid Copolymere (z.B. Gafquat® HS 100); Copolymere aus Vinylpyrrolidon und Dimethylaminoethylmethacrylat; Copolymere aus Vinylpyrrolidon, Vinylcaprolactam und Dimethylaminopropylacrylamid; Poly- oder Oligoester, aufgebaut aus mindestens einer ersten Monomerart, die ausgewählt ist aus mit mindestens einer quaternären Ammoniumgruppe substituierten Hydroxysäure; endständig mit quaternären Ammoniumgruppen substituierte Dimethylpolysiloxane.

Geeignete kationische Polymere, die von natürlichen Polymeren abgeleitet sind, sind insbesondere kationische Derivate von Polysacchariden, beispielsweise kationische Derivate von Cellulose, Stärke oder Guar. Geeignet sind weiterhin Chitosan und Chitosanderivate. Kationische Polysaccharide haben z.B. die allgemeine Formel

$$G\text{-}O\text{-}B\text{-}N^+R^aR^bR^c \qquad X^-$$

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
$R^a$, $R^b$ und $R^c$ sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in $R^a$, $R^b$ und $R^c$ vorzugsweise maximal 20 ist;
X ist ein übliches Gegenanion, beispielsweise ein Halogen, Acetat, Phosphat, Nitrat oder Alkylsulfat, vorzugsweise ein Chlorid. Kationische Cellulosen sind z.B. solche mit den INCI-Bezeichnungen Polyquaternium-10 oder Polyquaternium-24. Ein geeignetes kationisches Guarderivat hat z.B. die INCI-Bezeichnung Guar Hydroxypropyltrimonium Chloride.

Besonders bevorzugte kationaktive Stoffe sind Chitosan, Chitosansalze und Chitosanderivate. Bei den erfindungsgemäß einzusetzenden Chitosanen handelt es sich um vollständig oder partiell deacetylierte Chitine. Das Molekulargewicht kann über ein breites Spektrum verteilt sein, beispielsweise von 20.000 bis ca. 5 Millionen g/mol, z.B. von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein bevorzugtes Chitosansalz ist Chitosoniumpyrrolidoncarboxylat, z.B. Kytamer® PC mit einem Molekulargewicht von ca. 200.000 bis 300.000 g/mol und Deacetylierung von 70 bis 85%. Als Chitosanderivate kommen quaternierte, alkylierte oder hydroxyalkylierte Derivate, z.B. Hydroxyethyl-, Hydroxypropyl- oder Hydroxybutylchitosan in Betracht. Die Chitosane oder Chitosanderivate liegen vorzugsweise in neutralisierter oder partiell neutralisierter Form vor. Der Neutralisationsgrad liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Bevorzugte kationische Polymere auf natürlicher Basis: kationische Cellulosederivate aus Hydroxyethylcellulose und Diallyldimethylammoniumchlorid; kationische Cellulosederivate aus Hydroxyethylcellulose und mit Trimethylammonium substituiertem Epoxid; Chitosan und dessen Salze; Hydroxyalkylchitosane und deren Salze; Alkyl-hydroxyalkylchitosane und deren Salze; N-Hydroxyalkylchitosanalkylether; N-Hydroxyalkylchitosanbenzylether.

Geeignete nichtionische Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide; Polyvinylalkohole sowie Polyethylenglykol/Polypropylenglykol Copolymere. Geeignete natürliche filmbildende Polymere sind z.B. Cellulosederivate, z.B. Hydroxyalkylcellulose.

Bevorzugte nichtionische Polymere sind:
Polyvinylpyrrolidon, Polyvinylcaprolactam, Vinylpyrrolidon/Vinylacetat Copolymere, Polyvinylalkohol, Isobutylen/Ethylmaleimid/Hydroxyethylmaleimid Copolymer; Copolymyere aus Vinylpyrrolidon, Vinylacetat und Vinylpropionat.

Erfindungsgemäße, dauerhaft geschäumte Produkte, welche mindestens ein haarfestigendes Polymer enthalten zeichen sich außer durch die besonderen haptischen Eigenschaften und verbesserten Anwendungseigenschaften auch durch eine hervorragende, starke bis extra starke haarfestigende Wirkung aus.


Aufschäumung mit Gasen

In den erfindungsgemäßen Mitteln sind die Massen mit Luft oder einem Gas gemäß Anspruch 1 bis zu einem Aufschäumgrad von typischerweise mindestens 10% und bis zu 500%, vorzugsweise zwischen 20 und 200 %, besonders bevorzugt zwischen 30 und 100% stabil aufgeschäumt. Der Aufschäumgrad im Sinne der vorliegenden Erfindung stellt das Volumenverhältnis dar und berechnet sich aus den Dichten der Zusammensetzung vor und nach dem Aufschäumen folgendermaßen:

$$[(D_0/D)-1] * 100\%$$

wobei $D_0$ die Dichte vor Aufschäumen und D die Dichte nach Aufschäumen bezeichnet. Als Gase werden für das Aufschäumen neben Luft insbesondere Stickstoff, Kohlendioxid, Stickoxide, Edelgase oder Gemische der genannten Gase. Von besonderem Vorteil ist die Verwendung von inerten, sauerstofffreien Gasen wie Stickstoff oder Kohlendioxid zur Herstellung von aufgeschäumten Produken mit einem Gehalt an oxidationsempfindlichen Stoffen.

Die Begriffe 'dauerhaft aufgeschäumt' bzw. 'Permanentschaum' beziehen sich auf Produktmassen, die sich dadurch auszeichnen, dass in ihnen eine gasförmige Substanz in Form von Gasbläschen homogen verteilt ist und in dieser homogenen Verteilung über einen Zeitraum von mindestens einer Woche, vorzugsweise mindestens 1 Monat, besonders bevorzugt mindestens 6 Monaten bei Lagerung bei Raumtemperatur (20°C) erhalten bleibt, d.h. der Aufschäumgrad noch mindestens 10%, bevorzugt noch mindestens 20% beträgt. In den aufgeschäumten Produktmassen sind Gasblasen einer Größe von vorzugsweise zwischen 0,0001 und 10 mm, besonders bevorzugt zwischen 0,01 und 1 mm enthalten. Der mittlere Durchmesser der Gasblasen beträgt vorzugsweise von 0,1 bis 0,8 mm, besonders bevorzugt von 0,2 bis 0,4 mm. Die Dichte des erfindungsgemäßen Mittels ist durch Eintragen eines Gases in die zugrundeliegende Zusammensetzung auf vorzugsweise kleiner oder gleich 0,8 g/ml, insbesondere auf 0,2 bis 0,7 g/ml, besonders bevorzugt auf 0,2 bis 0,4 g/ml eingestellt.

Tenside

Ein besonderer erfindungsgemäßer Vorteil ist, dass es möglich ist, tensidfreie dauerhaft geschäumte Produkte herzustellen, d.h. ohne dass neben den obligatorischen Bestandteilen weitere oberflächenaktive Verbindungen enthalten sein müssen. Eine Ausführungsform der Erfindung betrifft daher Zusammensetzungen, die im wesentlichen frei sind von weiteren oberflächenaktiven Verbindungen, insbesondere frei von anionischen Tensiden. Dies führt zu besonders hautverträglichen Produkten. Die Produkte sind im wesentlichen frei von oberflächenaktiven Verbindungen, wenn entweder keine oder weniger als 1 Gew.% Tenside enthalten sind.

Selbstverständlich können aber für spezielle Anwendungsfälle zusätzlich Tenside enthalten sein. So können z.B. Haarpflegemittel haarpflegende kationische Tenside enthalten oder Körperreinigungsmittel können waschaktive anionische, amphotere oder nichtionische Tenside enthalten oder es können Emulgatoren zur Emulgierung oder Solubilisierung von Wirk- und Hilfsstoffen enthalten sein. Als Tenside sind nicht-ionische, anionische, kationische, amphotere oder zwitterionische Tenside geeignet. Geeignete Tenside sind beispielsweise die im 'International Cosmetic Ingredient Dictionary and Handbook', 7.Auflage, Band 2 im Abschnitt 'Surfactants', insbesondere im Unterabschnitt 'Surfactants - Emulsifying Agents' aufgeführten Tenside. Die Tenside können in Mengen von 0,1 bis 30 Gew.%, vorzugsweise von 0,2 bis 5 Gew.% bezogen auf die ungeschäumte Zusammensetzung vorliegen. Höhere Tensidmengen wie z.B. 5 bis 25 Gew.% sind enthalten, wenn das erfindungsgemäße Mittel als Haut- und Haarreinigungsmittel (Shampoo) verwendet wird.

Nichtionische Tenside sind z.B. oxethylierte Fettalkohole, oxethylierte Nonylphenole, Alkylpolyglycoside, Fettsäuremono- und -diglyceride, ethoxyliertes und hydriertes oder nicht hydriertes Rizinusöl, Fettsäurealkanolamide, oxethylierte Fettsäureester. Kationische Tenside sind z.B. langkettige quaternäre Ammoniumverbindungen wie sie unter den CTFA-Bezeichnungen "Quaternium" bekannt sind wie z.B. Alkyltrimethylammoniumsalze oder Dialkyldimethylammoniumsalze mit C8- bis C22-Alkylgruppen. Geeignete kationische Tenside sind solche der allgemeinen Formel

$$N^{(+)} R^1R^2R^3R^4 \qquad X^{(-)}$$

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit jeweils 1 bis 22 C-Atomen bedeuten, wobei mindestens einer der Reste R1 bis R4 mindestens 8 C-Atome aufweist und $X^{(-)}$ ein kosmetisch verträgliches Anion darstellt, z.B. ein Halogen, Acetat, Phosphat, Nitrat oder Alkylsulfat, vorzugsweise ein Chlorid. Die aliphatischen Gruppen können zusätzlich zu den Kohlenstoffatomen und den Wasserstoffatomen auch Querverbindungen oder andere Gruppen wie z.B. weitere Aminogruppen enthalten. Beispiele für geeignete kationische Tenside sind die Chloride oder Bromide von Alkyldimethylbenzylammoniumsalzen, Alkyltrimethylammoniumsalzen, z.B. Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, z.B. Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt ist Cetyltrimethylammoniumchlorid. Geeignete kationische Tenside sind insbesondere auch die sogenannten Esterquats, z.B. C8- bis C18-Alkylester von Betain, beispielsweise Palmitylbetainchlorid.

Geeignete anionische Tenside, insbesondere zur Verwendung in erfindungsgemäßen Haar- und Körperreinigungsmitteln sind ausgewählt aus Alkali- oder Erdalkalisalzen der C10- bis C18-Alkylsulfate, der C10- bis C18-Alkylsulfonate, der C10- bis C18-Alkylbenzolsulfonate, der C10- bis C18-Xylolsulfonate und der mit 1 bis 10 Ethylenoxideinheiten ethoxylierten C10- bis C18-Alkylethersulfate, den ethoxylierten Sulfobernsteinsäurehalbestern der allgemeinen Formel

$$R- (OCH_2CH_2)_m-O_2C- CH_2-CH (SO_3M) -COOM$$

wobei R einen C10- bis C18-Alkylrest bedeutet, M ein Alkali- oder Erdalkalikation darstellt und m eine Zahl von 1 bis 10 bedeutet und den Alkylethercarboxylaten der allgemeinen Formel

$$R- (OCH_2CH_2)_n-OCH_2-COOM$$

wobei R einen C10- bis C18-Alkylrest, M ein Alkali- oder Erdalkalikation und n eine Zahl von 1 bis 20 bedeutet, wobei die Alkali- und Erdalkalisalze der mit 1 bis 10 Ethylenoxideinheiten ethoxylierten C10- bis C18-Alkylethersulfate besonders bevorzugt sind.

Geeignete amphotere Tenside, insbesondere zur Verwendung in erfindungsgemäßen Haar- und Körperreinigungsmitteln sind ausgewählt aus Derivaten aliphatischer quaternärer Ammonium, Phosphonium- und Sulfoniumverbindungen der allgemeinen Formel

$$R^1\text{-}Y^{(+)}\,(R^2)\,_x\text{-}CH_2\text{-}R^3\text{-}Z^{(-)}$$

wobei R1 eine lineare oder verzweigte Alkyl-, Alkenyl-, oder Hydroxyalkylgruppe mit 8 bis 18 C-Atomen und 0 bis 10 Ethylenoxideinheiten und 0 bis 1 Glycerineinheit darstellt; Y eine N-, P- oder S-haltige Gruppe ist; R2 eine Alkyl oder Monohydroxyalkylgruppe mit 1 bis 3 C-Atomen ist; x gleich 1 ist, falls Y ein S-Atom ist und x gleich 2 ist, wenn Y ein N- oder ein P-Atom ist; R3 eine Alkyl- oder Hydroxyalkylgruppe mit 1 bis 4 C-Atomen ist und Z eine Carboxylat-, Sulfat-, Phosphonat- oder Phosphatgruppe darstellt. Weitere geeignete amphotere Tenside sind solche, die sich von Betain ableiten, z.B. C8- bis C18-Alkylbetaine wie Cocodimethylcarboxymethylbetain, Lauryldimethylcarboxymethylbetain, Lauryldimethylalpha-carboxyethylbetain, Cetyldimethylcarboxymethylbetain, Oleyldimethyl-gamma-carboxypropylbetain oder Lauryl-bis-(2-hydroxy-propyl)-alpha-carboxyethylbetain; C8- bis C18-Alkylsulfobetaine wie Cocodimethylsulfopropylbetain, Stearyldimethylsulfopropylbetain, Lauryldimethylsulfoethylbetain, Lauryl-bis-(2-hydroxyethyl)sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie z.B. das Kokosfettsäureamidopropylbetain (INCI-Bezeichnung: Cocamidopropylbetain) und das N-Kokosfettsäureamidoethyl-N-[2-(carboxymethoxy)ethyl]glycerin (INCI-Bezeichnung: Cocoamphocarboxyglycinate).

Gegenstand der Erfindung sind auch kosmetische, pharmazeutische oder dermatologische Mittel, enthaltend oder bestehend aus einer erfindungsgemäßen, dauerhaft geschäumten Zusammensetzung und zusätzlich enthaltend mindestens einen Wirkstoff, ausgewählt aus pharmazeutischen Wirkstoffen, dermatologischen Wirkstoffen, Hautpflegewirkstoffen, Haarpflegewirkstoffen, Dauerwellwirkstoffen, Haarfarbstoffen und Haarfarbstoffvorprodukten.

Bevorzugte Mittel weisen einen Gehalt auf an

(A) mindestens einer der oben genannten alkoxylierten Silikonverbindungen,
(B) gegebenenfalls mindestens einem Konsistenzgeber, ausgewählt aus bei 25°C wachsartig-festen Stoffen und Verdickern,
(C) Wasser und
(D) mindestens einem Wirkstoff, ausgewählt aus pharmazeutischen Wirkstoffen, dermatologischen Wirkstoffen, Hautpflegewirkstoffen, Haarpflegewirkstoffen, Dauerwellwirkstoffen, Haarfarbstoffen und Haarfarbstoffvorprodukten,

wobei das Mittel mit Luft oder einem inerten Gas aufgeschäumt ist und dauerhaft eine Dichte von kleiner oder gleich 0,8 g/cm³ aufweist.

Die zusätzlichen Wirkstoffe sind vorzugsweise in einer Menge von z.B. 0,01 bis 10 Gew.%, besonders bevorzugt von 0,1 bis 5 Gew.% bezogen auf das nicht geschäumte Mittel, enthalten. Die Wirkstoffe können z.B. ausgewählt sein aus Pflanzen- und Kräuterextrakten, Protein- und Seidenhydrolysaten, Lichtschutzmitteln, Antioxidantien, Radikalfängern, Antischuppenwirkstoffen, Glanzgebern, Vitaminen, Panthenol, Weichmachern, Kämmbarkeitsverbesserern, hauterweichenden, anfeuchtenden oder feuchthaltenden Stoffen, entzündungshemmenden Substanzen, Proteinen, Insektenrepellentien, Bakteriziden, Viruziden, antimikrobiell, proteolytisch oder keratolytisch wirksamen Substanzen, keratinreduzierenden Stoffen, Oxidationsstoffen, direktziehenden Haarfarbstoffen, Oxidationsfarbstoffvorstufen.

Geeignete haarpflegende Zusätze sind insbesondere Pflanzen- und Kräuterextrakte, Protein- und Seidenhydrolysate, Lichtschutzmittel, Antioxidantien, Radikalfänger, Antischuppenwirkstoffe, Glanzgeber, Vitamine, Panthenol, Weichmacher, Kämmbarkeitsverbesserer etc.. Als Kämmbarkeitsverbesserer kommen kationische oder kationaktive haarpflegende Stoffe in Frage, z.B. kationische Polymere, kationische Tenside, kationische Silikonverbindungen, kationisch derivatisierte Proteine oder Proteinhydrolysate und Betain mit jeweils mindestens einer kationischen oder kationaktiven Gruppe. Eine besondere Ausführungsform der Erfindung sind Haar- und Körperreinigungsmittel in Form eines stabil aufgeschäumten Produktes enthaltend die erfindungsgemäße Zusammensetzung und mindestens ein waschaktives Tensid.

Geeignete hautpflegende wirksame Stoffe sind z.B. weichmachende, anfeuchtende oder feuchthaltende Stoffe, entzündungshemmende Substanzen, Lichtschutzmittel, Vitamine, Proteine, Insektenrepellentien, Bakterizide, Viruzide, antimikrobiell, proteolytisch oder keratolytisch wirksame Substanzen, etc..

Erfindungsgemäße Haarfärbemittel können sowohl in Form eines oxidativen als auch nicht-oxidativen Färbemittels auf Basis von oxidativen und/oder direktziehenden Farbstoffen vorliegen. Die Gesamtmenge der in dem erfindungsgemäßen Mittel enthaltenen Oxidationsfarbstoffvorstufen beträgt etwa 0,01 bis 12 Gew.%, insbesondere etwa 0,2 bis 6 Gew.%. Als geeignete Oxidationsfarbstoffvorstufen können beispielsweise die in der WO 02/67882 genannten Entwicklersubstanzen und Kupplersubstanzen und mit sich selbst kuppelnden Verbindungen genannt werden. Die Gesamtmenge an direktziehenden Farbstoffen beträgt in dem erfindungsgemäßen Mittel etwa 0,01 bis 7 Gew.%, vorzugsweise etwa 0,2 bis 4 Gew.%. Für das erfindungsgemäße Mittel geeignet sind insbesondere die in der WO 02/67882 genannten direktziehenden Farbstoffe.

Eine besondere Ausführungsform der Erfindung betrifft ein Haarpflegemittel, welches zusätzlich mindestens einen haarpflegenden Wirkstoff enthält. Haarpflegemittel sind z.B. Conditioner, Treatments, Haarkuren, Spülungen oder dergleichen. Der haarpflegende Wirkstoff ist insbesondere ausgewählt aus kationischen Tensiden, aminsubstituierten Tensiden, kationischen Silikonverbindungen, aminsubstituierten Silikonverbindungen, kationischen Polymeren und aminsubstituierten Polymeren. Der haarpflegende Wirkstoff kann in Mengen zwischen 0,01 und 10,0 Gew.%, insbesondere zwischen 0,01 und 5,0 Gew.%, bezogen auf das fertige Produkt, eingesetzt werden. Das erfindungsgemäße Haarpflegemittel kann nach Applikation auf das trockene, feuchte oder nasse Haar entweder im Haar verbleiben oder es kann nach einer geeigneten Einwirkzeit wieder ausgespült werden. Die Einwirkzeiten hängen von der Art des Haares ab. Als allgemeine Richtlinie kann von Einwirkzeiten zwischen 0,5 und 30 Minuten, insbesondere zwischen 0,5 und 10 Minuten, vorzugsweise zwischen 1 und 5 Minuten ausgegangen werden. Insbesondere eine Kombination aus einem Amidoamin und/oder einem quaternisierten Amidoamin gemäß einer der unten aufgeführten allgemeinen Formeln und einem endfunktionaliserten, diquaternären Silikonpolymer ist in hervorragender Weise geeignet, ein kosmetisches Haarpflegemittel mit hervorragenden, verbesserten haarpflegenden Wirkungen bereitzustellen.

Als haarpflegende Wirkstoffe geeignete kationische bzw. aminsubstituierte Polymere sind u.a. die oben genannten Polymere. Neben den oben genannten kationischen Tensiden sind weitere geeignete kationische oder aminsubstituierte Tenside solche der Formel R1-NH-(CH$_2$)n-NR2R3 oder der Formel

$$R1\text{-}NH\text{-}(CH_2)n\text{-}N^+R2R3R4 \ X^-$$

worin R1 ein Acyl- oder ein Alkylrest mit 8 bis 24 C-Atomen ist, welcher verzweigt oder unverzweigt, gesättigt oder ungesättigt sein kann, wobei der Acyl- und/oder der Alkylrest eine oder mehrere OH-Gruppen enthalten kann, R2, R3 und R4 unabhängig voneinander Wasserstoff, Alkyl- oder Alkoxyalkylreste mit 1 bis 6 C-Atomen sind, welche gleich oder verschieden, gesättigt oder ungesättigt und mit einer oder mehreren Hydroxygruppen substituiert sein können, X$^-$ ein Anion ist, insbesondere ein Halogenidion oder eine Verbindung der allgemeinen Formel RSO$_3$- , worin R die Bedeutung von gesättigten oder ungesättigten Alkylresten mit 1 bis 4 C-Atomen hat, und n eine ganze Zahl zwischen 1 und 10, vorzugsweise von 2 bis 5 bedeutet.

Vorzugsweise ist der haarpflegende wirkstoff ein Amidoamin und/oder ein quaternisiertes Amidoamin der oben genannten Formeln, worin R1 ein verzweigter oder unverzweigter, gesättigter oder ungesättigter Acylrest mit 8 bis 24 C-Atomen ist, welcher mindestens eine OH-Gruppe enthalten kann. Bevorzugt sind auch solche Amine und/oder quaternisierte Amine, in denen mindestens einer der Reste R2, R3 und R4 ein Rest gemäß der allgemeinen Formel CH$_2$CH$_2$OR5 bedeuten, worin R5 die Bedeutung von Alkylresten mit 1 bis 4 C-Atomen, Hydroxyethyl oder H haben kann.

Geeignete Amine oder Amidoamine, welche gegebenenfalls quaternisiert sein können, sind z.B. solche mit den INCI-Bezeichnungen Ricinoleamidopropyl Betaine, Ricinoleamidopropyl Dimethylamine, Ricinoleamidopropyl Dimethyl Lactate, Ricinoleamidopropyl Ethyldimonium Ethosulfate, Ricinoleamidopropyltrimonium Chloride, Ricinoleamidopropyltrimonium methosulfate, Cocamidopropyl Betaine, Cocamidopropyl Dimethylamine, Cocamidopropyl Ethyldimonium Ethosulfate, Cocamidopropyltrimonium Chloride, Behenamidopropyl Dimethylamine, Isostearylamidopropyl Dimethylamine, Stearylamidopropyl Dimethylamine, Quaternium-33, Undecyleneamidopropyltrimonium Methosulfate.

Geeignete kationaktive Silikonverbindungen weisen vorzugsweise entweder mindestens eine primäre, sekundäre oder tertiäre Aminogruppe (aminsubstituierte Silikonverbindung) oder mindestens eine quaternäre Ammoniumgruppe (kationische Silikonverbindung) auf. Geeignete Silikonpolymere mit Aminogruppen sind unter der INCI-Bezeichnung Amodimethicone oder PEG-x Amodimethicone bekannt, wobei x den Ethoxylierungsgrad angibt, welcher vorzugsweise zwischen 2 und 10 liegt. Hierbei handelt es sich um Polydimethylsiloxane mit seiten- oder endständigen Aminoalkylgruppen. Geeignete Aminosilikone sind solche der allgemeinen Formel

$$R^8R^9R^{10}Si\text{-} (OSiR^{11}R^{12}) \ x\text{-} (OSiR^{13}Q) \ \gamma\text{-}OSiR^{14}R^{15}R^{16}$$

R$^8$, R$^9$, R$^{14}$ und R$^{15}$ sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;

R$^{10}$ und R$^{16}$ sind unabhängig voneinander gleich oder verschieden und bedeuten -(CH$_2$)$_a$-NH$_2$ mit a gleich 1 bis 6, C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;

R$^{11}$, R$^{12}$ und R$^{13}$ sind unabhängig voneinander gleich oder verschieden und bedeuten Wasserstoff, C1- bis C20-Kohlenwasserstoff, welcher O- und N-Atome enthalten kann, vorzugsweise C1- bis C10-Alkyl oder Phenyl, besonders bevorzugt C1- bis C4-Alkyl, insbesondere Methyl;

Q bedeutet -A-NR$^{17}$R$^{18}$, oder -A-N$^+$R$^{17}$R$^{18}$R$^{19}$ wobei A für eine divalente C1- bis C20-Alkylenverbindungsgruppe steht, welche auch O- und N-Atome sowie OH-Gruppen enthalten kann, und

R$^{17}$, R$^{18}$ und R$^{19}$ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind (CH$_2$) )-NH$_2$,- (CH$_2$)$_3$NHCH$_2$CH$_2$NH$_2$, -(CH$_2$)$_3$OCH$_2$CHOHCH$_2$NH$_2$ und - (CH$_2$)$_3$N(CH$_2$CH$_2$OH)$_2$, -(CH$_2$)$_3$-NH$_3$$^+$ und -(CH$_2$)$_3$OCH$_2$CHOHCH$_2$N$^+$(CH$_3$)$_2$R$^{20}$, wobei R$^{20}$ ein C1- bis C22-Alkylrest ist, der auch OH-Gruppen aufweisen kann. X bedeutet eine Zahl zwischen 1 und 10.000, vorzugsweise zwischen 1 und 1.000;

Y bedeutet eine Zahl zwischen 1 und 500, vorzugsweise zwischen 1 und 50.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5.

Geeignete Silikonpolymere mit zwei endständigen quaternären Ammoniumgruppen sind unter der INCI-Bezeichnung Quaternium-80 bekannt. Hierbei handelt es sich um Dimethylsiloxane mit zwei endständigen Aminoalkylgruppen. Geeignete quaternäre Aminosilikone sind solche der allgemeinen Formel

$$R^{21}R^{22}R^{23}N^+-A-SiR^8R^9-(OSiR^{11}R^{12})_n-OSiR^8R^9-A-N^+R^{21}R^{22}R^{23} \ 2X^-$$

A steht für eine divalente C1- bis C20-Alkylenverbindungsgruppe, welche auch O- und N-Atome sowie OH-Gruppen enthalten kann, und ist vorzugsweise - (CH$_2$)$_3$OCH$_2$CHOHCH$_2$N$^+$(CH$_3$)$_2$R$^{20}$,

wobei R$^{21}$ ein C1- bis C22-Alkylrest ist, der auch OH-Gruppen aufweisen kann;

R$^8$, R$^9$, R$^{11}$ und R$^{12}$ haben die gleiche Bedeutung wie oben und sind vorzugsweise Methyl;

R$^{21}$, R$^{22}$, und R$^{23}$ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen;

n ist eine Zahl von 0 bis 200, vorzugsweise von 10 bis 100. Geeignete Produkte sind z.B. Abil® Quat 3270, 3272 und 3274.

Herstellung

Erfindungsgemäße Zusammensetzungen und Mittel können hergestellt werden durch (A) Vorlegen einer Zusammensetzung, enthaltend mindestens eine der oben genannten alkoxylierten Silikonverbindungen, gegebenenfalls mindestens einen Konsistenzgeber, ausgewählt aus bei 25°C wachsartig-festen Stoffen und Verdickern und Wasser und (B) gegebenenfalls anschließend oder gleichzeitig Erwärmen der Zusammensetzung auf eine Temperatur oberhalb der Schmelztemperatur der wachsartigen Stoffe, falls ein wachsartiger Stoff enthalten ist und (C) anschließend oder gleichzeitig Aufschlagen oder Aufschäumen der Zusammensetzung mit Luft und/oder einem Gas gemäß Anspruch 1.

Die Herstellung von dispersionsförmigen, ungeschäumten Zusammensetzungen kann nach einem an sich bekannten Verfahren erfolgen. Eine Übersicht über moderne Verfahren zur Herstellung von halbfesten und flüssigen Emulsionen ist dem Artikel im SÖFW-Journal, 124. Jahrgang, 5/98, Seiten 308 bis 313 sowie dem Artikel im SÖFW-Journal, 118. Jahrgang, 5/92, Seiten 287 bis 296 zu entnehmen. Die Herstellung erfolgt in der Regel in der Weise, dass die hydrophobe Fettphase auf ca. 75°C erhitzt und mit der ebenfalls ca. 75°C heißen hydrophilen Wasserphase unter intensiver Vermischung mit einem Mixer oder Homogenisator vereinigt wird. Anschließend erfolgt eine Abkühlung der fertigen Dispersion. Der Gaseintrag erfolgt vorzugsweise zu einem Zeitpunkt, zu dem die Mischung noch nicht vollständig abgekühlt ist, z.B. bei 30-40°C.

Das Aufschlagen oder Aufschäumen kann mittels einer hierfür geeigneten Vorrichtung erfolgen, z.B. mittels eines schnell laufenden Rührwerk, wobei das Gas der umgebenden Atmosphäre (vorzugsweise Luft) in die Masse eingetragen wird. Eine zunächst heiße, flüssige Mischung verfestigt sich beim Abkühlen. Das Aufschäumen kann auch erfolgen, indem die flüssige, ungeschäumte Zusammensetzung durch einen Mischer, welcher eine Fördereinrichtung, einen Mischkopf und einen zusätzlichen Anschluss zur Zuführung eines Gases aufweist, geleitet wird und gleichzeitig dem Mischer über den zusätzlichen Anschluss ein Gas zugeführt wird. Die Zusammensetzung kann in einem Aufschlaggerät (z.B. Euromix oder dem dynamischen Schaumgenerator Top Mix von Hansa Industrie Mixer oder mit einem Ultra Turax-Labormischer) mit einem Gas, vorzugsweise Luft, CO$_2$ oder Stickstoff aufgeschlagen werden. Besonders vorteilhaft ist die Verwendung von Stickstoff bei oxidationsempfindlichen Inhaltsstoffen. Der Aufschlag liegt vorzugsweise zwischen 20 und 200%, wobei über den Aufschlag das Gefüge und die Konsistenz beeinflußt und wunschgemäß eingestellt werden kann. Bevorzugt sind Mischer bestehend aus einer Fördereinrichtung und einem Rotor/Stator-Mischkopf, wobei während des Mischvorgangs dem Rotor/Stator-Mischkopf über einen zusätzlichen Anschluß ein Gas zugeführt werden kann. Besonders bevorzugt ist ein Becomix Duohomogenisator, welcher einen zusätzlichen Anschluß aufweist, durch den das Gas direkt auf den Rotor aufgegeben werden kann und erst in den Zahnkränzen des Homogenisators mit der vorgelegten Phase in Berührung kommt. Hierbei kann das Gas mittels Anlegens eines Unterdrucks (Vakuums)

am Rührkessel und/oder mittels eines Überdrucks in der Gaszuführleitung zugeführt werden. Durch Veränderung der Fördermenge, Drehzahl, Temperatur, Druck und zugeführter Gasmenge lassen sich Schaumdichte, Konsistenz, Viskosität und Schaumblasengröße gezielt einstellen.

Die stabil aufgeschäumten Produkte werden in geeignete Verpackungen wie z.B. Tiegel oder Tuben abgefüllt. Die Verpackungen sind vorteilhafterweise aus einem durchsichtigen Material wie z.B. Glas oder einem durchsichtigen Kunststoff, um die vorteilhaften optischen Eigenschaften erkennbar zu machen. Die Verpackungen können auch Vorrichtungen zur Produktabgabe aufweisen, insbesondere mechanisch betriebene Pumpvorrichtungen. Die Permanentschäume sind über längere Zeit lagerstabil und fühlen sich je nach Schaumblasengröße creme- bis sahneartig oder samtig an.

Konsistenz, Viskosität

Bei dem Endprodukt handelt es sich um eine viskose Zusammensetzung, für die sich die gewünschte Endviskosität in der Regel bereits nach Abkühlen ergibt. Die gewünschte Endviskosität kann aber auch durch Zugabe eines Elektrolyten, ausgewählt aus Alkali- oder Erdalkalisalzen wie z.B. Natriumchlorid oder Natriumsulfat oder eines anderen, verdickend wirkenden Stoffes wie Cellulosen oder Cellulosederivaten eingestellt werden.

Für eine besonders gute Stabilität der geschäumten Produkte sollten sowohl die ungeschäumte wie auch die geschäumte Zusammensetzung eine Fließgrenze und eine ausreichend hohe Viskosität aufweisen. Die Viskosität der ungeschäumten Zusammensetzung liegt vorzugsweise im Bereich von 1000 bis 30000 mPa s, besonders bevorzugt im Bereich von 3000 bis 20000 mPa s und die Viskosität der geschäumten Zusammensetzung liegt vorzugsweise zwischen 1000 und 100000 mPa s, besonders bevorzugt zwischen 1500 und 80000 mPa s gemessen mit dem Viskosimeter Haake VT 550, Meßsystem SVDIN bei 25°C und einem Schergefälle von 50 $s^{-1}$. Die Fließgrenze beträgt vorzugsweise mindestens 10 Pa, besonders bevorzugt mindestens 50 Pa, gemessen mit dem Bohlin Rheometer CS, Meßsystem CP 4/40 bei 25°C mit linear steigender Scherspannung von 0,1 bis 600 Pa (200 s).

Die erfindungsgemäßen geschäumten Produkte zeichnen sich durch einen Gehalt an sehr kleinen, homogen verteilten Gasblasen aus. Die Mittel weisen besonders vorteilhafte sensorische Eigenschaften auf:

- sie haben einen sehr angenehmen haptischen Eindruck und fühlen sich cremig oder samtig, aber nicht klebrig an, sie haben eine geringe Dichte, fühlen sich leicht an und sind leicht auf keratinischen Oberflächen wie Haut oder Haaren zu verteilen;
- sie weisen eine angenehme, kosmetische, weiße Farbe besonderer Reinheit auf; insbesondere läßt sich das Aussehen einer in ungeschäumten Zustand beigefarbenen oder gelbstichigen Creme verbessern, da schon geringe Mengen feinst verteilter Luftbläschen die Creme weisser aussehen lassen;
- es sind akustische Effekte wahrnehmbar, z.B. ein Knistern beim Auspressen der Schäume aus Tuben bzw. beim Verteilen auf der Haut oder beim Einarbeiten in das Haar.

Die Mittel weisen weiterhin den Vorteil auf, dass sie auch auf schrägen Flächen gut zu applizieren sind, nicht ablaufen und nicht tropfen.

Bevorzugte Ausführungsformen sind:

- eine permanent geschäumte kosmetische Hautcreme, enthaltend mindestens einen hautpflegenden Stoff
- eine permanent geschäumte Sonnencreme, enthaltend mindestens einen organischen oder anorganischen UV-Filter
- eine permanent geschäumte Haarstylingcreme, enthaltend mindestens einen haarfestigenden Stoff
- eine permanent geschäumte Haarkurcreme, enthaltend mindestens einen haarpflegenden Stoff
- eine permanent geschäumte Dauerwell- oder Haarglättungscreme, enthaltend mindestens einen Dauerwell- bzw. Haarglättungswirkstoff
- eine permanent geschäumte Haarfärbereme, enthaltend mindestens einen direktziehenden Haarfarbstoff oder mindestens eine Oxidationsfarbstoffvorstufe.

Die nachfolgenden Beispiele sollen den Gegenstand der Erfindung näher erläutern.

**Beispiele**

**Beispiel 1: Dauerhaft geschäumtes Haarstylingprodukt**

| | |
|---|---|
| 3,5 g | Bis-PEG-12 Dimethicone Candelillate (Siliconyl Candelilla) |
| 2,0 g | Bis-PEG-20 Dimethicone (SF 1388, GE Silicones) |
| 1,5 g | Lanolinalkohol (Dusoran®) |
| 1,5 g | Vinylacetat/Crotonsäure/Vinylneodecanoat Copolymer (Resyn 28-2930, National Starch) |
| 1 g | kappa-Carrageenan (Genugel® X-901-02) |
| 0,5 g | Bienenwachs |
| 0,25 g | Aminomethylpropanol |
| 0,001 g | Acid Red 52 (CI 45100) |
| 10 g | Ethanol |
| 40 g | Wasser |

Die Stoffe werden zusammengegeben und auf ca. 90°C erhitzt. Die heiße Mischung wird dann mit einem schnelllaufenden Rührwerk aufgeschlagen und verfestigt sich beim Abkühlen. Zur Beschleunigung kann aktiv gekühlt werden. Man erhält einen festen Schaum (Dichte ca. 0.2-0.3g/ml), der in der Konsistenz an festes *Mousse au chocolat* erinnert. Er ist sahnig-cremig und knistert beim Verreiben auf der Hand. Trocknet der Schaum ein, so bleibt das Volumen der Masse zu ca. 90% erhalten und man erhält einen schaumig-gummiartigen, nicht-klebrigen Festkörper. Im verschlossenen Glas behält der Schaum sein Volumen und seine Eigenschaften auch über vier Wochen Lagerung bei 40°C. Man beobachtet lediglich eine geringe Schrumpfung der Masse um ca. 5-10%, die nach einigen Tagen beendet ist. Die Verreibbarkeit der feuchten Masse ist sehr leicht, ohne aufzuschäumen oder zu krümeln. Man erhält einen cremigen, farblos-durchsichtigen Film auf der Hand. Bei Anwendung auf dem Haar ist die Haarfestigung stark bis extra stark. Der Schaum eignet sich sehr gut zur Lockendefinition und Volumenerhöhung der Frisur.

## Beispiel 2: Dauerhaft geschäumtes Haarpflegeprodukt

| | |
|---|---|
| 3,5 g | Bis-PEG-12 Dimethicone Candelillate (Siliconyl Candelilla, Koster Keunen) |
| 0,5 g | Bis-PEG-12 Dimethicone Beeswax (Siliconyl Beeswax, Koster Keunen) |
| 2,0 g | Bis-PEG-20 Dimethicone (SF 1388, GE Silicones) |
| 1,5 g | Lanolinalkohol (Dusoran®) |
| 2 g | Cetearylalkohol (Lanette® 0) |
| 1 g | Stearamidopropyl Dimethylamine (Tegoamid® S18) |
| 1 g | kappa-Carrageenan (Genugel® X-901-02) |
| 1 g | PEG-7 Amodimethicone (Ultrasil® A-21, Noveon) |
| 0,001 g | Acid Red 52 (CI 45100) |
| 10 g | Ethanol |
| 40 g | Wasser |

## Beispiel 3: Dauerhaft geschäumtes Haarglättungsprodukt

| | |
|---|---|
| 3,5 g | Bis-PEG-12 Dimethicone Candelillate (Siliconyl Candelilla) |
| 2,0 g | Bis-PEG-20 Dimethicone (SF 1388, GE Silicones) |
| 1,5 g | Lanolinalkohol (Dusoran®) |
| 1 g | iota-Carrageenan (Genuvisco® X-904-02) |
| 0,5 g | Bienenwachs |
| 2,5 g | Ammoniak, 25%ig |
| 10 g | Ammoniumthioglycolat, 70%ig (ATG) |
| 4 g | Diammoniumdithioglycolat, 40%ig (DADTG) |
| 0,001 g | Acid Red 52 (CI 45100) |

(fortgesetzt)

| 10 g | Ethanol |
| 40 g | Wasser |

Das Carrageenan wird in heissem Wasser unter Zugabe von Bis-PEG-20 Dimethicone gelöst und langsam mit ATG und DADTG versetzt. Die geschmolzenen Wachse werden untergerührt und restliche Bestandteile zugefügt. Die warme Mischung wird dann mit einem schnelllaufenden Rührwerk aufgeschlagen und verfestigt sich beim Abkühlen. Zur Beschleunigung kann aktiv gekühlt werden. Man erhält einen weichen, elastischen Schaum (Dichte ca. 0.7 g/ml), der in der Konsistenz an *Mousse au chocolat* erinnert.

**Beispiel 4: Dauerhaft geschäumtes Haarfärbeprodukt**

| 3,5 g | Bis-PEG-12 Dimethicone Candelillate (Siliconyl Candelilla) |
| 2,0 g | Bis-PEG-20 Dimethicone (SF 1388, GE Silicones) |
| 1,5 g | Lanolinalkohol (Dusoran®) |
| 1 g | kappa-Carrageenan (GenugeL® X-901-02) |
| 0,5 g | Bienenwachs |
| 0,25 g | Aminomethylpropanol |
| 0,3 g | Rubinrot Y [1] |
| 0,15 g | 2-Amino-6-chloro-4-nitrophenol (Rodol® 9R Base) |
| 10 g | Ethanol |
| 40 g | Wasser |

[1] Mischung aus 70 Gew.% HC Red No. 10 (3-[(4-Amino-2-chloro-5-nitrophenyl)amino]1,2-propandiol) und 30 Gew.% HC Red No. 11 (3,3'-[(2-Chloro-5-nitro-1,4-phenylen)diimino]bis-1,2-propandiol)

Weglassen der erfindungsgemäß einzusetzenden alkoxylierten Silikonverbindungen oder deren Ersatz durch nicht erfindungsgemäße alkoxylierte Silikonverbindungen in den Beispielen 1 bis 4 führte nicht zu hinreichend stabilen Schaumprodukten.

In den vorstehenden Beispielen kann das Bis-PEG-20-Dimethicone jeweils durch Bis-PEG-18 Methyl Ether Dimethyl Silane (Dow Corning® 2501 Cosmetic Wax) ersetzt werden

**Patentansprüche**

1. Aufgeschäumte Zusammensetzung enthaltend

(A) mindestens eine alkoxylierte Silikonverbindung, welche zu einer oder mehreren der folgenden Verbindungsklassen gehört:

- bis-alkoxylierte Silikonverbindungen
- alkoxylierte Silikonwachse
- Ester aus Fettsäuren und alkoxylierten Silikonverbindungen
- wasserunlöslichen alkoxylierten Silikonverbindungen,

(B) mindestens einen Konsistenzgeber, ausgewählt aus bei 25°C wachsartig-festen Stoffen und Verdickern und
(C) Wasser,

wobei die Zusammensetzung mit einem Gas aufgeschäumt ist, welches ausgewählt ist aus Luft, Stickstoff, Kohlendioxid, Stickoxiden, Edelgasen oder einem Gemisch dieser Gase und dauerhaft eine Dichte von kleiner oder gleich 0,8 g/cm$^3$ aufweist, wobei der Aufschäumgrad nach Lagerung von mindestens einer Woche bei Raumtemperatur (20°C) noch mindestens 10% beträgt.

2. Zusammensetzung nach Anspruch 1, **dadurch gekennzeichnet, dass** die alkoxylierte Silikonverbindung in einer Menge von 0,1 bis 30 Gew.%, bezogen auf die ungeschäumte Wachsdispersion, enthalten ist und ausgewählt ist aus Blockcopolymeren mit einem Mittelblock aus Polydimethylsiloxan und Endblöcken aus Polyethylenoxid und/oder Polypropylenoxid; Fettsäureestern dieser Blockcopolymere und ethoxylierten Dimethylsilanmethylethern.

3. Zusammensetzung nach einem der vorhergehenden Ansprüche, **dadurch gekennzeichnet, dass** die alkoxylierte Silikonverbindung ausgewählt ist aus Bis-(polyethylenoxid)-polydimethylsiloxan und Fettsäureestern von Bis-(polyethylenoxid)polydimethylsiloxan oder deren Gemisch.

4. Zusammensetzung nach einem der vorhergehenden Ansprüche, **dadurch gekennzeichnet, dass** der wachsartige Feststoff (B) in einer Menge von 0,05 bis 50 Gew.%, bezogen auf die ungeschäumte Zusammensetzung, enthalten ist, und ausgewählt ist aus tierischen Wachsen, pflanzlichen Wachsen, mineralischen Wachsen, synthetischen Wachsen, mikrokristallinen Wachsen, makrokristallinen Wachsen, Paraffinwachsen, Ozokerit, Montanwachsen, Fischer-Tropsch-Wachsen, Polyolefinwachsen, Silikonwachsen, Bienenwachs, Wollwachs, Wollwachsalkoholen, Candelillawachs, Carnaubawachs, Japanwachs, Fetten, Fettsäureestern, Fettsäureglyceriden, langkettigen C10- bis C22-Carbonsäuren, langkettigen C10- bis C22-Alkoholen, jeweils mit Schmelz- bzw. Erstarrungspunkten oberhalb 25°C.

5. Zusammensetzung nach einem der vorhergehenden Ansprüche, **dadurch gekennzeichnet, dass** als Komponente (B) sowohl mindestens ein wachsartiger Feststoff als auch mindestens ein Verdicker enthalten ist.

6. Zusammensetzung nach einem der vorhergehenden Ansprüche, **dadurch gekennzeichnet, dass** der Verdicker in einer Menge von 0,05 bis 30 Gew.%, bezogen auf die ungeschäumte Zusammensetzung, enthalten ist und ausgewählt ist aus

   - synthetischen Polymeren, ausgewählt aus Polyvinylpyrrolidon und vernetzten Polyacrylaten;
   - Polymeren auf natürlicher Basis, ausgewählt aus Sclerotium Gum, Stärke, Gelatine, Cellulose, Carboxymethylcellulose, Hydroxypropylcellulose, Methylcellulose, Hydroxypropylmethylcellulose, Hydroxyethylcellulose, mikrokristalline Cellulose, Agar-agar, Carrageenanen, Alginate, Carouba Gum, Guar Gum, alkyliertes Guar, hydroxyalkyliertes Guar, Karaya Gum, Xanthan Gum, Gum arabicum und Pektin;
   - anorganischen Verdickern, ausgewählt aus Hectorite, Bentonite, Aluminiumsilikaten, Magnesiumsilikaten.

7. Zusammensetzung nach Anspruch 6, **dadurch gekennzeichnet, dass** der Verdicker Carrageenan ist und in einer Menge von 0,5 bis 10 Gew.%, bezogen auf die ungeschäumte Zusammensetzung, enthalten ist.

8. Zusammensetzung nach einem der vorhergehenden Ansprüche, **dadurch gekennzeichnet, dass** zusätzlich mindestens ein haarfestigendes und/oder haarpflegendes Polymer enthalten ist.

9. Zusammensetzung nach Anspruch 8, **dadurch gekennzeichnet, dass** das haarfestigende und/oder haarpflegende Polymer in einer Menge von 0,01 bis 20 Gew.%, bezogen auf die ungeschäumte Zusammensetzung, enthalten ist und ausgewählt ist aus

   - Polymeren mit anionischen oder anionisierbaren Gruppen, ausgewählt aus Terpolymeren aus Acrylsäure, Ethylacrylat und N-tert-Butylacrylamid; vernetzten oder unvernetzten Vinylacetat/Crotonsäure Copolymeren; Terpolymeren aus tert.-Butylacrylat, Ethylacrylat und Methacrylsäure; Natriumpolystyrolsulfonat; Copolymeren aus Vinylacetat, Crotonsäure und Vinylpropionat; Copolymeren aus Vinylacetat, Crotonsäure und Vinylneodecanoat; Aminomethylpropanol-Acrylat Copolymeren; Copolymeren aus Vinylpyrrolidon und mindestens einem weiteren Monomer ausgewählt aus Acrylsäure, Methacrylsäure, Acrylsäureestern und Methacrylsäureestern; Copolymeren aus Methylvinylether und Maleinsäuremonoalkylestern; Aminomethylpropanolsalze von Copolymeren aus Allylmethacrylat und mindestens einem weiteren Monomer ausgewählt aus Acrylsäure, Methacrylsäure, Acrylsäureestern und Methacrylsäureestern; vernetzten Copolymeren aus Ethylacrylat und Methacrylsäure; Copolymeren aus Vinylacetat, Mono-n-butylmaleat und Isobornylacrylat; Copolymeren aus zwei oder mehr Monomeren ausgewählt aus Acrylsäure, Methacrylsäure, Acrylsäureestern und Methacrylsäureestern, Copolymeren aus Octylacrylamid und mindestens einem Monomeren ausgewählt aus Acrylsäure, Methacrylsäure, Acrylsäureestern und Methacrylsäureestern; Polyestern aus Diglycol, Cyclohexandimethanol, Isophtalsäure und Sulfoisophtalsäure;

- Polymeren mit kationischen oder kationisierbaren Gruppen, ausgewählt aus kationischen Cellulosederivaten aus Hydroxyethylcellulose und Diallyldimethylammoniumchlorid; kationischen Cellulosederivaten aus Hydroxyethylcellulose und mit Trimethylammonium substituiertem Epoxid; Poly(dimethyldiallylammoniumchlorid); Copolymeren aus Acrylamid und Dimethyldiallylammoniumchlorid; quaternären Ammoniumpolymeren, gebildet durch die Reaktion von Diethylsulfat und einem Copolymer aus Vinylpyrrolidon und Dimethylaminoethylmethacrylat; quaternären Ammoniumpolymeren aus Methylvinylimidazoliumchlorid und Vinylpyrrolidon; Polyquaternium-35; Polymer aus Trimethylammoniumethyl-methacrylatchlorid; Polyquaternium-57; endständig mit quaternären Ammoniumgruppen substituierte Dimethylpolysiloxane; Copolymer aus Vinylpyrrolidon, Dimethylaminopropylmethacrylamid und Methacryloylaminopropyllauryldimethylammoniumchlorid; Chitosan und dessen Salze; Hydroxyalkylchitosane und deren Salze; Alkyl-hydroxyalkylchitosane und deren Salze; N-Hydroxyalkylchitosanalkylether; N-Hydroxyalkylchitosanbenzylether; Copolymer aus Vinylcaprolactam, Vinylpyrrolidon und Dimethylaminoethylmethacrylat; Copolymeren aus Vinylpyrrolidon und Dimethylaminoethylmethacrylat, Copolymeren aus Vinylpyrrolidon, Vinylcaprolactam und Dimethylaminopropylacrylamid; Poly- oder Oligoestern, aufgebaut aus mindestens einer ersten Monomerart, die ausgewählt ist aus mit mindestens einer quaternären Ammoniumgruppe substituierten Hydroxysäure; Terpolymeren aus Vinylpyrrolidon, Methacrylamid und Vinylimidazol;
- zwitterionischen und/oder amphoteren Polymeren, ausgewählt aus Copolymeren aus Octylacrylamid, Acrylsäure, Butylaminoethylmethacrylat, Methylmethacrylat und Hydroxypropylmethacrylat; Copolymeren aus Laurylacrylat, Stearylacrylat, Ethylaminoxidmethacrylat und mindestens einem Monomeren ausgewählt aus Acrylsäure, Methacrylsäure, Acrylsäureestern und Methacrylsäureestern; Copolymeren aus Methacryloylethylbetain und mindestens einem Monomeren ausgewählt aus Methacrylsäure und Methacrylsäureestern; Copolymeren aus Acrylsäure, Methylacrylat und Methacrylamidopropyltrimethylammoniumchlorid; Oligomeren oder Polymeren, herstellbar aus quaternären Crotonbetainen oder quaternären Crotonbetainestern;
- nichtionischen Polymeren, ausgewählt aus Polyvinylpyrrolidon, Polyvinylcaprolactam, Vinylpyrrolidon/ Vinylacetat Copolymeren, Polyvinylalkohol, Isobutylen/ Ethylmaleimid/Hydroxyethylmaleimid Copolymer; Copolymeren aus Vinylpyrrolidon, Vinylacetat und Vinylpropionat.

**10.** Zusammensetzung nach einem der vorhergehenden Ansprüche, **dadurch gekennzeichnet, dass** sie im wesentlich frei ist von oberflächenaktiven anionischen Verbindungen.

**11.** Zusammensetzung nach einem der vorhergehenden Ansprüche, **dadurch gekennzeichnet, dass** sie hergestellt ist durch

(A) Vorlegen einer Zusammensetzung, enthaltend mindestens eine alkoxylierte Silikonverbindung gemäß Anspruch 1 und Wasser,
(B) gegebenenfalls anschließend oder gleichzeitig Erwärmen der Zusammensetzung auf eine Temperatur oberhalb der Schmelztemperatur von enthaltenen wachsartigen Stoffen
(C) und anschließend oder gleichzeitig Aufschlagen oder Aufschäumen der Zusammensetzung mit Luft und/ oder einem inerten Gas.

**12.** Kosmetisches Mittel, enthaltend oder bestehend aus der aufgeschäumten Zusammensetzung gemäß einem der Ansprüche 1 bis 11 und zusätzlich enthaltend mindestens einen Wirkstoff, ausgewählt aus Hautpflegewirkstoffen, Haarpflegewirkstoffen, Dauerwellwirkstoffen, Haarfarbstoffen und Haarfarbstoffvorprodukten.

**13.** Mittel nach Anspruch 12, **dadurch gekennzeichnet, dass** der Wirkstoff ausgewählt ist aus Pflanzen- und Kräuterextrakten, Protein- und Seidenhydrolysaten, Lichtschutzmitteln, Antioxidantien, Radikalfängern, Antischuppenwirkstoffen, Glanzgebern, Vitaminen, Panthenol, Weichmachern, Kämmbarkeitsverbesserern, hauterweichenden, anfeuchtenden oder feuchthaltenden Stoffen, entzündungshemmenden Substanzen, Proteinen, Insektenrepellentien, Bakteriziden, Viruziden, antimikrobiell, proteolytisch oder keratolytisch wirksamen Substanzen, keratinreduzierenden Stoffen, Oxidationsstoffen, direktziehenden Haarfarbstoffen, Oxidationsfarbstoffvorstufen.

**14.** Mittel nach Anspruch 12 oder 13 in Form

- einer permanent geschäumten kosmetischen Hautcreme, enthaltend mindestens einen hautpflegenden Stoff
- einer permanent geschäumten Sonnencreme, enthaltend mindestens einen organischen oder anorganischen UV-Filter
- einer permanent geschäumten Haarstylingcreme, enthaltend mindestens einen haarfestigenden Stoff
- einer permanent geschäumten Haarpflegecreme, enthaltend mindestens einen haarpflegenden Stoff,

- einer permanent geschäumten Dauerwell- oder Haarglättungscreme, enthaltend mindestens einen Dauerwell- bzw. Haarglättungswirkstoff
- einer permanent geschäumten Haarfärbereme, enthaltend mindestens einen direktziehenden Haarfarbstoff oder mindestens eine Oxidationsfarbstoffvorstufe.

15. Haarpflegemittel enthaltend eine Zusammensetzung gemäß einem der Ansprüche 1 bis 11 und zusätzlich mindestens einen Haarpflegewirkstoff, ausgewählt aus kationischen Tensiden, aminsubstituierten Tensiden, kationischen Silikonverbindungen, aminsubstituierten Silikonverbindungen, kationischen Polymeren und aminsubstituierten Polymeren.

16. Verwendung einer in einer geeigneten Verpackung vorliegenden, dauerhaft aufgeschäumten Zusammensetzung nach einem der Ansprüche 1 bis 11 oder eines Mittels nach einem der Ansprüche 12 bis 15 zur kosmetischen Haarpflege, zum Haarstylen, zur Haar- oder Körperreinigung oder zur kosmetischen Hautpflege.

17. Verwendung einer in einer geeigneten Verpackung vorliegenden, dauerhaft aufgeschäumten Zusammensetzung nach einem der Ansprüche 1 bis 11 oder eines Mittels nach einem der Ansprüche 12 bis 15 zur Herstellung einer permanent geschäumten kosmetischen Hautcreme, enthaltend mindestens einen hautpflegenden Stoff; einer permanent geschäumten Sonnencreme, enthaltend mindestens einen organischen oder anorganischen UV-Filter; einer permanent geschäumten Haarstylingcreme, enthaltend mindestens einen haarfestigenden Stoff; einer permanent geschäumten Haarkurcreme, enthaltend mindestens einen haarpflegenden Stoff; oder einer permanent geschäumter Dauerwell- oder Haarglättungscreme, enthaltend mindestens einen Dauerwell- bzw. Haarglättungswirkstoff.

18. Verfahren zur Herstellung einer Zusammensetzung nach einem der Ansprüche 1 bis 11 oder eines Mittels nach einem der Ansprüche 12 bis 15 durch

(A) Vorlegen einer Zusammensetzung, enthaltend mindestens eine alkoxylierte Silikonverbindung gemäß Anspruch 1 und Wasser,
(B) gegebenenfalls anschließend oder gleichzeitig Erwärmen der Zusammensetzung auf eine Temperatur oberhalb der Schmelztemperatur von enthaltenen wachsartigen Stoffen
(C) und anschließend oder gleichzeitig Aufschlagen oder Aufschäumen der Zusammensetzung mit Luft und/ oder einem inerten Gas.

19. Verfahren nach Anspruch 18, **dadurch gekennzeichnet, dass** das Aufschlagen mittels einem schnell laufenden Rührwerk erfolgt.

20. Verfahren nach Anspruch 18, **dadurch gekennzeichnet, dass** das Aufschäumen erfolgt indem die flüssige, ungeschäumte Zusammensetzung durch einen Mischer, welcher eine Fördereinrichtung, einen Mischkopf und einen zusätzlichen Anschluss zur Zuführung eines Gases aufweist, geleitet wird und gleichzeitig dem Mischer über den zusätzlichen Anschluss ein Gas zugeführt wird.

**Claims**

1. Foamed composition comprising

(A) at least one alkoxylated silicone compound which belongs to one or more of the following compound classes:

- bisalkoxylated silicone compounds
- alkoxylated silicone waxes
- esters of fatty acids and alkoxylated silicone compounds
- water-insoluble alkoxylated silicone compounds,

(B) at least one consistency regulator selected from thickeners and substances that are waxy-solid at 25°C and
(C) water,

where the composition is foamed with a gas which is selected from air, nitrogen, carbon dioxide, nitric oxides, noble gases or a mixture of these gases and durably has a density of less than or equal to 0.8 g/cm$^3$, where the degree of foaming following storage for at least one week at room temperature (20°C) is still at least 10%.

**2.** Composition according to Claim 1, **characterized in that** the alkoxylated silicone compound is present in an amount of from 0.1 to 30% by weight, based on the unfoamed wax dispersion, and is selected from block copolymers with a middle block of polydimethylsiloxane and end blocks of polyethylene oxide and/or polypropylene oxide; fatty acid esters of these block copolymers and ethoxylated dimethylsilane methyl ethers.

**3.** Composition according to one of the preceding claims, **characterized in that** the alkoxylated silicone compound is selected from bis(polyethylene oxide) polydimethylsiloxane and fatty acid esters of bis(polyethylene oxide) polydimethylsiloxane or mixture thereof.

**4.** Composition according to one of the preceding claims, **characterized in that** the waxy solid (B) is present in an amount of from 0.05 to 50% by weight, based on the unfoamed composition, and is selected from animal waxes, vegetable waxes, mineral waxes, synthetic waxes, microcrystalline waxes, macrocrystalline waxes, paraffin waxes, ozocerite, montan waxes, Fischer-Tropsch waxes, polyolefin waxes, silicone waxes, beeswax, wool wax, wool wax alcohols, candelilla wax, carnauba wax, Japan wax, fats, fatty acid esters, fatty acid glycerides, long-chain C10- to C22-carboxylic acids, long-chain C10- to C22-alcohols, in each case with melting or solidification points above 25°C.

**5.** Composition according to one of the preceding claims, **characterized in that** both at least one waxy solid and also at least one thickener is present as component (B).

**6.** Composition according to one of the preceding claims, **characterized in that** the thickener is present in an amount of from 0.05 to 30% by weight, based on the unfoamed composition, and is selected from

- synthetic polymers selected from polyvinylpyrrolidone and crosslinked polyacrylates;
- natural-based polymers selected from sclerotium gum, starch, gelatine, cellulose, carboxymethylcellulose, hydroxypropylcellulose, methylcellulose, hydroxypropylmethylcellulose, hydroxyethylcellulose, microcrystalline cellulose, agar-agar, carrageenans, alginates, carouba gum, guar gum, alkylated guar, hydroxyalkylated guar, karaya gum, xanthan gum, gum Arabic and pectin;
- inorganic thickeners selected from hectorites, bentonites, aluminium silicates, magnesium silicates.

**7.** Composition according to Claim 6, **characterized in that** the thickener is carrageenan and is present in an amount of from 0.5 to 10% by weight, based on the unfoamed composition.

**8.** Composition according to one of the preceding claims, **characterized in that** at least one hair-setting and/or hair care polymer is additionally present.

**9.** Composition according to Claim 8, **characterized in that** the hair-setting and/or hair care polymer is present in an amount of from 0.01 to 20% by weight, based on the unfoamed composition, and is selected from

- polymers with anionic or anionizable groups, selected from terpolymers of acrylic acid, ethyl acrylate and N-tert-butylacrylamide; crosslinked or uncrosslinked vinyl acetate/crotonic acid copolymers; terpolymers of tert-butyl acrylate, ethyl acrylate and methacrylic acid; sodium polystyrenesulphonate; copolymers of vinyl acetate, crotonic acid and vinylpropionate; copolymers of vinyl acetate, crotonic acid and vinylneodecanoate; aminomethylpropanol acrylate copolymers; copolymers of vinylpyrrolidone and at least one further monomer selected from acrylic acid, methacrylic acid, acrylic acid esters and methacrylic acid esters; copolymers of methyl vinyl ether and maleic acid monoalkyl esters; aminomethylpropanol salts of copolymers of allyl methacrylate and at least one further monomer selected from acrylic acid, methacrylic acid, acrylic acid esters and methacrylic acid esters; crosslinked copolymers of ethyl acrylate and methacrylic acid; copolymers of vinyl acetate, mono-n-butyl maleate and isobornyl acrylate; copolymers of two or more monomers selected from acrylic acid, methacrylic acid, acrylic acid esters and methacrylic acid esters, copolymers of octylacrylamide and at least one monomer selected from acrylic acid, methacrylic acid, acrylic acid esters and methacrylic acid esters; polyesters of diglycol, cyclohexanedimethanol, isophthalic acid and sulphoisophthalic acid;
- polymers with cationic and cationizable groups selected from cationic cellulose derivatives of hydroxyethylcellulose and diallyldimethylammonium chloride; cationic cellulose derivatives of hydroxyethylcellulose and epoxide substituted with trimethylammonium; poly(dimethyldiallylammonium chloride); copolymers of acrylamide and dimethyldiallylammonium chloride; quaternary ammonium polymers formed by the reaction of diethyl sulphate and a copolymer of vinylpyrrolidone and dimethylaminoethyl methacrylate; quaternary ammonium polymers of methylvinylimidazolium chloride and vinylpyrrolidone; polyquaternium-35; polymer of trimethylammonium ethyl methacrylate chloride; polyquaternium-57; dimethylpolysiloxanes terminally substituted with quaternary ammonium groups; copolymer of vinylpyrrolidone, dimethylaminopropylmethacrylamide and methacryloylaminopropyllauryldimethylammonium chloride; chitosan and salts thereof; hydroxyalkylchitosans and salts thereof; alkylhydroxyalkylchitosans and salts thereof; N-hydroxyalkylchitosan alkyl ethers; N-hydroxyalkylchitosan benzyl ethers; copolymer of vinylcaprolactam, vinylpyrrolidone and dimethylaminoethyl methacrylate; copolymers of vinylpyrrolidone and dimethylaminoethyl methacrylate, copolymers of vinylpyrrolidone, vinylcaprolactam and dimethylaminopropylacrylamide; poly- or oligoesters composed of at least one first type of monomer which is selected from hydroxy acid substituted with at least one quaternary ammonium group; terpolymers of vinylpyrrolidone, methacrylamide and vinylimidazole;

- zwitterionic and/or amphoteric polymers selected from copolymers of octylacrylamide, acrylic acid, butylaminoethyl methacrylate, methyl methacrylate and hydroxypropyl methacrylate; copolymers of lauryl acrylate, stearyl acrylate, ethylamine oxide methacrylate and at least one monomer selected from acrylic acid, methacrylic acid, acrylic acid esters and methacrylic acid esters; copolymers of methacryloylethylbetaine and at least one monomer selected from methacrylic acid and methacrylic acid esters; copolymers of acrylic acid, methyl acrylate and methacrylamidopropyltrimethylammonium chloride; oligomers or polymers preparable from quaternary croton betaines or quaternary croton betaine esters;

- nonionic polymers selected from polyvinylpyrrolidone, polyvinylcaprolactam, vinylpyrrolidone/vinyl acetate copolymers, polyvinyl alcohol, isobutylene/ethylmaleimide/hydroxyethylmaleimide copolymer; copolymers of vinylpyrrolidone, vinyl acetate and vinylpropionate.

10. Composition according to one of the preceding claims, **characterized in that** it is essentially free from surface-active anionic compounds.

11. Composition according to one of the preceding claims, **characterized in that** it is prepared by

(A) initially introducing a composition comprising at least one alkoxylated silicone compound according to Claim 1 and water,
(B) optionally subsequently or simultaneously heating the composition to a temperature above the melting temperature of waxy substances that are present
(C) and subsequently or simultaneously impacting or foaming the composition with air and/or an inert gas.

12. Cosmetic agent comprising or consisting of the foamed composition according to one of Claims 1 to 11 and additionally comprising at least one active ingredient selected from skincare active ingredients, hair care active ingredients, permanent wave active ingredients, hair dyes and hair dye precursors.

13. Agent according to Claim 12, **characterized in that** the active ingredient is selected from plant and herb extracts, protein and silk hydrolysates, photoprotectants, antioxidants, free-radical scavengers, antidandruff active ingredients, shine-imparting agents, vitamins, panthenol, softeners, combability improvers, skin-softening, moisturizing or humectant substances, antiinflammatory substances, proteins, insect repellents, bactericides, virucides, antimicrobially, proteolytically or keratolytically effective substances, keratin-reducing substances, oxidation substances, direct hair dyes, oxidation dye precursors.

14. Agent according to Claim 12 or 13 in the form

- of a permanently foamed cosmetic skin cream comprising at least one skincare substance,
- of a permanently foamed sun cream comprising at least one organic or inorganic UV filter
- of a permanently foamed hairstyling cream comprising at least one hair-setting substance
- of a permanently foamed hair care cream comprising at least one hair care substance,
- of a permanently foamed permanent wave or hair smoothing cream comprising at least one permanent wave or hair smoothing active ingredient
- of a permanently foamed hair colouring cream comprising at least one direct hair dye or at least one oxidation dye precursor.

15. Hair care agent comprising a composition according to one of Claims 1 to 11 and additionally at least one hair care active ingredient selected from cationic surfactants, amine-substituted surfactants, cationic silicone compounds, amine-substituted silicone compounds, cationic polymers and amine-substituted polymers.

16. Use of a durable foamed composition according to one of Claims 1 to 11 or of an agent according to one of Claims 12 to 15 present in a suitable packaging for cosmetic hair care, for hairstyling, for hair or body cleansing or for cosmetic skincare.

**17.** Use of a durable foamed composition according to one of Claims 1 to 11 or of an agent according to one of Claims 12 to 15 present in a suitable packaging for the preparation of a permanently foamed cosmetic skin cream comprising at least one skincare substance; of a permanently foamed sun cream comprising at least one organic or inorganic UV filter; of a permanently foamed hairstyling cream comprising at least one hair setting substance; of a permanently foamed hair treatment cream comprising at least one hair care substance; or of a permanently foamed permanent wave or hair smoothing cream comprising at least one permanent wave or hair smoothing active ingredient.

**18.** Process for the preparation of a composition according to one of Claims 1 to 11 or of an agent according to one of Claims 12 to 15 by

(A) initially introducing a composition comprising at least one alkoxylated silicone compound according to Claim 1 and water,
(B) optionally subsequently or simultaneously heating the composition to a temperature above the melting temperature of waxy substances that are present
(C) and subsequently or simultaneously impacting or foaming the composition with air and/or an inert gas.

**19.** Process according to Claim 18, **characterized in that** the impacting takes place by means of a highspeed stirrer.

**20.** Process according to Claim 18, **characterized in that** the foaming takes place by passing the liquid unfoamed composition through a mixer which has a conveying device, a mixing head and an additional connection for supplying a gas, and simultaneously supplying a gas to the mixer via the additional connection.

## Revendications

**1.** Composition transformée en mousse, contenant

(A) au moins un composé silicone alcoxylé qui appartient à une ou plusieurs des classes de composés suivants :

- composés silicone bis-alcoxylés
- cires de silicone alcoxylées
- esters d'acides gras et de composés silicone alcoxylés,
- composés silicone alcoxylés insolubles dans l'eau,

(B) au moins un agent de consistance choisi parmi des substances solides-cireuses à 25 °C et des épaississants et
(C) de l'eau,

la composition étant transformée en mousse avec un gaz qui est choisi parmi l'air, l'azote, le dioxyde de carbone, les oxydes d'azote, les gaz rares ou un mélange de ces gaz et présentant durablement une densité inférieure ou égale à 0,8 g/cm$^3$, le degré de moussage étant encore d'au moins 10 % après stockage pendant au moins une semaine à la température ambiante (20 °C).

**2.** Composition selon la revendication 1, **caractérisée en ce que** le composé silicone alcoxylé est contenu en une quantité de 0,1 à 30 % en poids, par rapport à la dispersion de cire transformée en mousse et est choisi parmi des copolymères séquencés comportant une séquence médiane de polydiméthylsiloxane et des séquences terminales de polyoxyéthylène et/ou polyoxypropylène ; des esters d'acides gras de ces copolymères séquencés et des éthers méthyliques de diméthylsilane éthoxylés.

**3.** Composition selon l'une quelconque des revendications précédentes, **caractérisée en ce que** le composé silicone alcoxylé est choisi parmi le bis(polyoxyéthylène)-polydiméthylsiloxane et des esters d'acides gras de bis(polyoxyéthylène)-polydiméthylsiloxane ou un mélange de ceux-ci.

**4.** Composition selon l'une quelconque des revendications précédentes, **caractérisée en ce que** le solide cireux (B) est contenu en une quantité de 0,05 à 50 % en poids, par rapport à la composition transformée en mousse, et est choisi parmi des cires animales, des cires végétales, des cires minérales, des cires synthétiques, des cires microcristallines, des cires macrocristallines, des cires de paraffine, l'ozokérite, les cires de lignite, les cires de Fischer-Tropsch, les cires polyoléfiniques, les cires de silicone, la cire d'abeille, la lanoline, les alcolanums, la cire de candelilla, la cire de carnauba, la cire du Japon, des graisses, des esters d'acides gras, des glycérides d'acides gras, des acides carboxyliques à longue chaîne en $C_{10}$-$C_{22}$, des alcools à longue chaîne en $C_{10}$-$C_{22}$, ayant chacun un point de fusion ou de solidification supérieur à 25 °C.

5. Composition selon l'une quelconque des revendications précédentes, **caractérisée en ce qu'**aussi bien au moins un solide cireux qu'au moins un épaississant est contenu en tant que composant (B).

6. Composition selon l'une quelconque des revendications précédentes, **caractérisée en ce que** l'épaississant est contenu en une quantité de 0,05 à 30 % en poids, par rapport à la composition transformée en mousse, et est choisi parmi

   - des polymères synthétiques, choisis parmi le polyvinylpyrrolidone et des polyacrylates réticulés ;
   - des polymères à base naturelle, choisis parmi la gomme de sclérotium, l'amidon, la gélatine, la cellulose, la carboxyméthyl-cellulose, l'hydroxypropylcellulose, la méthylcellulose, l'hydroxypropylméthyl-cellulose, l'hydroxyéthylcellulose, la cellulose microcristalline, l'agar-agar, le carraghénane, les alginates, la gomme carouba, la gomme guar, le guar alkylé, le guar hydroxyalkylé, la gomme karaya, la gomme xanthane, la gomme arabique et la pectine ;
   - des épaississants inorganiques, choisis parmi les hectorites, les bentonites, les silicates d'aluminium, les silicates de magnésium.

7. Composition selon la revendication 6, **caractérisée en ce que** l'épaississant est le carraghénane et est contenu en une quantité de 0,5 à 10 % en poids, par rapport à la composition transformée en mousse.

8. Composition selon l'une quelconque des revendications précédentes, **caractérisée en ce qu'**en outre est contenu au moins un polymère fixant les cheveux et/ou pour le soin des cheveux.

9. Composition selon la revendication 8, **caractérisée en ce que** le polymère fixant les cheveux et/ou pour le soin des cheveux est contenu en une quantité de 0,01 à 20 % en poids, par rapport à la composition transformée en mousse, et est choisi parmi

   - des polymères à groupes anioniques ou pouvant être rendus anioniques, choisis parmi des terpolymères d'acide acrylique, acrylate d'éthyle et N-tert-butylacrylamide ; des copolymères acétate de vinyle/acide crotonique réticulés ou non réticulés ; des terpolymères d'acrylate de tert-butyle, acrylate d'éthyle et acide méthacrylique ; le polystyrènesulfonate de sodium ; des copolymères d'acétate de vinyle, acide crotonique et propionate de vinyle ; des copolymères d'acétate de vinyle, acide crotonique et néodécanoate de vinyle ; des copolymères aminométhylpropanol/acrylate ; des copolymères de vinylpyrrolidone et d'au moins un autre monomère choisi parmi l'acide acrylique, l'acide méthacrylique, des esters d'acide acrylique et des esters d'acide méthacrylique ; des copolymères d'oxyde de méthyle et de vinyle et de maléates de monoalkyle ; des sels avec l'aminométhylpropanol de copolymères de méthacrylate d'allyle et d'au moins un autre monomère choisi parmi l'acide acrylique, l'acide méthacrylique, des esters d'acide acrylique et des esters d'acide méthacrylique ; des copolymères réticulés d'acrylate d'éthyle et d'acide méthacrylique ; des copolymères d'acétate de vinyle, maléate de mono-n-butyle et acrylate d'isobornyle ; des copolymères de deux ou plus de deux monomères choisis parmi l'acide acrylique, l'acide méthacrylique, les esters d'acide acrylique et les esters d'acide méthacrylique ; des copolymères d'octylacrylamide et d'au moins un monomère choisi parmi l'acide acrylique, l'acide méthacrylique, les esters d'acide acrylique et les esters d'acide méthacrylique ; des polyesters de diglycol, cyclohexanediméthanol, acide isophtalique et acide sulfo-isophtalique ;
   - des polymères à groupes cationiques ou pouvant être rendus cationiques, choisi parmi des dérivés cationiques de cellulose à base d'hydroxyéthylcellulose et chlorure de diallyldiméthylammonium ; des dérivés cationiques de cellulose à base d'hydroxyéthylcellulose et d'époxyde substitué par triméthylammonium ; le poly(chlorure de diméthyldiallylammonium) ; des copolymères d'acrylamide et chlorure de diméthyldiallylammonium ; des polymères d'ammonium quaternaire formés par la réaction du sulfate de diéthyle et d'un copolymère de vinylpyrrolidone et méthacrylate de diméthylaminoéthyle ; des polymères d'ammonium quaternaire à base de chlorure de méthylvinylimidazolium et vinylpyrrolidone ; le Polyquaternium-35 ; un polymère de chlorure de triméthylammoniumméthacrylate d'éthyle ; le Polyquaternium-57 ; des diméthylpolysiloxanes substitués en bout de chaîne par des groupes ammonium quaternaires ; un copolymère de vinylpyrrolidone, diméthylaminopropylméthacrylamide et chlorure de méthacryloylaminopropyllauryldiméthylammonium ; le chitosane et ses sels ;

des hydroxyalkylchitosanes et leurs sels ; des alkyl-hydroxyalkylchitosanes et leurs sels ; des éthers alkyliques de N-hydroxyalkylchitosane ; des éthers benzyliques de N-hydroxyalkylchitosane ; un copolymère de vinylcaprolactame, vinylpyrrolidone et méthacrylate de diméthylaminoéthyle ; des copolymères de vinylpyrrolidone et méthacrylate de diméthylaminoéthyle ; des copolymères de vinylpyrrolidone, vinylcaprolactame et diméthylaminopropylacrylamide ; des poly- ou oligoesters formés à partir d'au moins un premier type de monomère qui est choisi parmi des acides hydroxylés substitués par au moins un groupe ammonium quaternaire ; des terpolymères de vinylpyrrolidone, méthacrylamide et vinylimidazole ;

- des polymères zwitterioniques et/ou des polymères amphotères, choisis parmi des copolymères d'octylacrylamide, acide acrylique, méthacrylate de butylaminoéthyle, méthacrylate de méthyle et méthacrylate d'hydroxypropyle ; des copolymères d'acrylate de lauryle, acrylate de stéaryle, aminooxyméthacrylate d'éthyle et d'au moins un monomère choisi parmi l'acide acrylique, l'acide méthacrylique, des esters d'acide acrylique et des esters d'acide méthacrylique ; des copolymères de méthacryloyléthylbétaïne et d'au moins un monomère choisi parmi l'acide méthacrylique et des esters d'acide méthacrylique ; des copolymères d'acide acrylique, acrylate de méthyle et chlorure de méthacrylamidopropyltriméthylammonium ; des oligomères ou polymères pouvant être préparés à partir de crotonobétaïnes quaternaires ou d'esters de crotonobétaïnes quaternaires ;

- des polymères non ioniques, choisis parmi la polyvinylpyrrolidone, le polyvinylcaprolactame, des copolymères vinylpyrrolidone/acétate de vinyle, le poly(alcool vinylique) un copolymère isobutylène/éthylmaléimide/ hydroxyéthylmaléimide ; des copolymères de vinylpyrrolidone, acétate de vinyle et propionate de vinyle.

10. Composition selon l'une quelconque des revendications précédentes, **caractérisée en ce qu'**elle est pratiquement exempte de composés tensioactifs anioniques.

11. Composition selon l'une quelconque des revendications précédentes, **caractérisée en ce qu'**elle est préparée par

(A) disposition au préalable d'une composition contenant au moins un composé silicone alcoxylé selon la revendication 1 et de l'eau,
(B) éventuellement chauffage simultané ou subséquent de la composition jusqu'à une température supérieure à la température de fusion des substances cireuses contenues et
(C) fouettement ou transformation en mousse, simultané(e) ou subséquent(e), de la composition avec de l'air et/ou un gaz inerte.

12. Produit cosmétique, contenant ou consistant en la composition transformée en mousse selon l'une quelconque des revendications 1 à 11 et en outre contenant au moins une substance active choisie parmi des substances actives pour le soin de la peau, des substances actives pour le soin des cheveux, des substances actives pour l'ondulation permanente, des colorants pour cheveux et des précurseurs de colorants pour cheveux.

13. Produit selon la revendication 12, **caractérisé en ce que** la substance active est choisie parmi des extraits de plantes et d'herbes aromatiques, des hydrolysats de protéine et de soie, des photoprotecteurs, des antioxydants, des capteurs de radicaux, des actifs antipelliculaires, des agents lustrants, des vitamines, le panthénol, des assouplissants, des agents améliorant l'aptitude au passage du peigne, des assouplissants pour la peau, des substances hydratantes ou retenant l'humidité, des substances anti-inflammatoires, des protéines, des insectifuges, des bactéricides, des virucides, des substances à action antimicrobienne, protéolytique ou kératolytique, des substances réduisant la kératine, des oxydants, des colorants directs pour cheveux, des précurseurs de colorants d'oxydation.

14. Produit selon la revendication 12 ou 13, sous forme

- d'une crème cosmétique pour la peau, transformée en mousse de façon permanente, contenant au moins une substance pour le soin de la peau,
- d'une crème solaire transformée en mousse de façon permanente, contenant au moins un filtre UV organique ou inorganique,
- d'une crème coiffante transformée en mousse de façon permanente, contenant au moins une substance fixant les cheveux,
- d'une crème pour le soin des cheveux, transformée en mousse de façon permanente, contenant au moins une substance de soin capillaire,
- d'une crème pour ondulation permanente ou pour lisser les cheveux, transformée en mousse de façon permanente, contenant au moins une substance active pour ondulation permanente ou lissage des cheveux,
- d'une crème de teinture pour cheveux, transformée en mousse de façon permanente, contenant au moins un colorant direct pour cheveux ou au moins un précurseur de colorant d'oxydation.

**15.** Produit de soin capillaire contenant une composition selon l'une quelconque des revendications 1 à 11 et en outre au moins une substance active pour le soin des cheveux, choisie parmi des tensioactifs cationiques, des tensioactifs substitués par des amines, des composés silicone cationiques substitués par des amines, des polymères cationiques et des polymères substitués par des amines.

**16.** Utilisation d'une composition transformée en mousse de façon durable, selon l'une quelconque des revendications 1 à 11, présente dans un emballage approprié, ou d'un produit selon l'une quelconque des revendications 12 à 15, pour le soin cosmétique des cheveux, pour le coiffage, pour le nettoyage du corps ou des cheveux ou pour le soin cosmétique de la peau.

**17.** Utilisation d'une composition transformée en mousse de façon durable, selon l'une quelconque des revendications 1 à 11, présente dans un emballage approprié, ou d'un produit selon l'une quelconque des revendications 12 à 15, pour la fabrication d'une crème cosmétique pour la peau, transformée en mousse de façon permanente, contenant au moins une substance pour le soin de la peau ; d'une crème solaire transformée en mousse de façon permanente, contenant au moins un filtre UV organique ou inorganique ; d'une crème coiffante transformée en mousse de façon permanente, contenant au moins une substance fixant les cheveux ; d'une crème pour le soin des cheveux, transformée en mousse de façon permanente, contenant au moins une substance de soin capillaire ; ou d'une crème pour ondulation permanente ou pour lisser les cheveux, transformée en mousse de façon permanente, contenant au moins une substance active pour ondulation permanente ou lissage des cheveux.

**18.** Procédé pour la préparation d'une composition selon l'une quelconque des revendications 1 à 11 ou d'un produit selon l'une quelconque des revendications 12 à 15, par

(A) disposition au préalable d'une composition contenant au moins un composé silicone alcoxylé selon la revendication 1 et de l'eau,
(B) éventuellement chauffage simultané ou subséquent de la composition jusqu'à une température supérieure à la température de fusion des substances cireuses contenues et
(C) fouettement ou transformation en mousse, simultané(e) ou subséquent(e), de la composition avec de l'air et/ou un gaz inerte.

**19.** Procédé selon la revendication 18, **caractérisé en ce que** le fouettement s'effectue au moyen d'un système d'agitation rapide.

**20.** Procédé selon la revendication 18, **caractérisé en ce qu'**on effectue la transformation en mousse en faisant passer la composition non transformée en mousse dans un mélangeur qui comporte un dispositif d'alimentation, une tête de mélange et un raccord supplémentaire pour l'apport d'un gaz et on envoie en même temps un gaz dans le mélangeur, par le raccord supplémentaire.

**EP 1 588 692 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 838210 A2
- EP 1078626 A2
- EP 1369104 A1
- WO 03028676 A2
- EP 1149872 A2
- WO 0241847 A
- WO 0267882 A
- EP 1046387 A
- EP 1216682 A
- CH 674804
- JP 56079613 A

**In der Beschreibung aufgeführte Nicht-Patentliteratur**

- *SÖFW-Journal,* Mai 1998, vol. 124, 308-313
- *SÖFW-Journal,* Mai 1992, vol. 118, 287-296